# EUROPEAN PATENT APPLICATION

(11) **EP 2 103 596 A1**
(43) Date of publication of application: **23.09.2009**
(21) Application number: 08384003.3
(22) Date of filing: 18.03.2008
(51) Int. Cl.: C07C 211/00, C07D 295/00, C07C 251/00

(54) **Process for the preparation of N-(phenylethyl) anilines salts and solvates thereof useful as serotonin 5-HT6 antagonists**

(71) Applicant: Laboratorios Del. Dr. Esteve, S.A., 08041 Barcelona (ES)
(72) Inventor: Sicre González, Cristina, 36203 Vigo (ES); Caamaño Moure, Ana María, 15705 Santiago de Compostela (ES); Torrens Jover, Antonio, 08221 Terrassa (ES)
(74) Representative: Ponti Sales, Adelaida

(57) **Abstract**

The invention relates to a process for preparing N-(1-phenylethyl)anilines, salts, and solvates thereof, to novel intermediates, and to the use of the intermediates in the preparation of serotonin 5-HT6 antagonists.

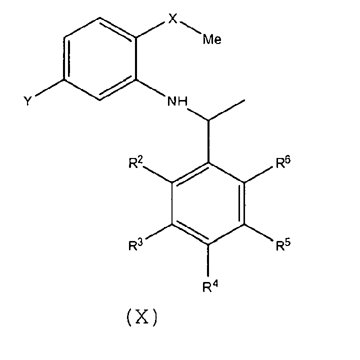

## Description

### Field of the invention

The invention relates to a process for preparing N-(1-phenylethyl)anilines, salts, and solvates thereof, to novel intermediates, and to the use of the intermediates in the preparation of serotonin 5-HT6 antagonists.

### Background of the invention

Multiple serotonin (5-hydroxytryptamine or 5-HT) receptor subtypes have been identified. Of particular interest is the 5-HT-6 serotonin receptor, which human gene was cloned and characterized by Kohen et al. (J. Neurochem. 66: 47-56, 1996). The 5-HT6 receptor (5-HT6R) stimulates adenylyl cyclase via G(s) coupling and regulates several neurotransmitter systems including glutamate, aspartate and acetylcholine.

5-HT6Rs have been mainly localized in olfactory tubercles, striatum, nucleus accumbens, and hippocampus with lower levels also found in amygdala and hypothalamus. 5-HT6 mRNA appears to be almost exclusively present in the brain with little evidence for its presence in peripheral tissues. Therefore, 5-HT6 antagonism has been proposed as a promising approach for treating cognitive impairment associated with neuropsychiatric disorders (e.g., Alzheimer's disease, bipolar affective disorders, Parkinson, schizophrenia) without having potential peripheral side effects.

As such, compounds with enhanced affinity and selectivity for the 5-HT6 receptor represent a real potential for treating cognitive dysfunction. Several 5-HT6R antagonists have been identified in a number of studies, e.g., by Isaac, M. et al. (2000) Bioorganic & Medicinal Chemistry Letters 10: 1719-1721); and in patent publications such as WO00/34242, W099/37623, WO99/42465, and WO99/02502.

Of particular interest are the compounds described in WO06/081332 that have 5-HT6 receptor antagonist activity and are believed to be of useful in treating or preventing obesity and type II diabetes, as well as in the treatment or prophylaxis of central nervous system disorders such as anxiety, depression, panic attacks, memory disorders, sleep disorders, binge disorders, migraine, anorexia, bulimia, obsessive compulsive disorders, psychoses, Alzheimer' s disease, Parkinson's disease, Huntington's chorea and/or schizophrenia, drug abuse, and Attention Deficit/Hyperactive Disorders (ADHD).

Amongst those compounds disclosed in WO06/081332, Compound A ([1-(5-Chloro-2,3-dimethoxy-phenyl)-ethyl]-(2-methanesulfonyl-5-piperazin-1-yl-phenyl)-amine hydrochloride salt), has been found to be a highly selective (>100-fold safety margin to more than 70 tested receptors) 5-HT6 receptor antagonist (K = 4nM), with excellent bioavailability in dogs (F=8 1%) and half-life (T1/2=5 h) following oral administration (5 mg/kg). Compound A also showed no significant CYP inhibition (IC₅₀ > 7.5 mM for CYP1A2, CYP3A4, CYP2C9 and CYP2C19; IC₅₀ = 7.8 µM for CYP2D6) and possesses excellent stability (HLM = > 90 minutes). It is reported that n *in vivo* studies, Compound A induces a significant reduction in food intake and body weight in rats and mice following sub-chronic dosing (10 and 30 mg/kg qd, i.p.).

According to the general synthetic route and the procedure described in Example 25 of the above mentioned patent application, Compound A may be prepared by reacting a phenylethyl amine with the appropriate substituted benzene in the presence of the base *N,N-*diisopropylethylamine, and the acetonitrile or DMF solvent, followed by the reaction of the purified resulting product with piperazine in the presence of the same base.

It is an objective of the present invention to provide an alternative synthetic route for the preparation of Compound A and *N*-(1-phenylethyl)anilines in general, as well as the salts and solvates thereof.

It is an objective of the present invention to provide an alternative synthetic route for the preparation of Compound A and *N*-(1-phenylethyl)anilines in general, as well as the salts and solvates thereof, wherein said route can improve at least one or more of the following process related parameters, i.e., purity, yield, simplification of the synthetic route, use one-pot procedures, use of affordable conditions, use of environmental-friendly conditions, use of non-toxic reagents, improved processability of the reagents, up-scalable process.

In one of the several attempts to design and test an optimized synthetic procedure for the industrial production of Compound A and *N*-(1-phenylethyl)anilines in general, the inventors have surprisingly found that said compounds can be prepared by reacting a phenylamine with a desired substituted benzaldehyde, resulting in the corresponding N-benzylideneaniline, which can be further reacted with a methyl nucleophile, resulting thereby into the sought core structure of *N*-(1-phenylethyl)anilines.

N-benzylideneaniline is a particularly useful reagent as it contributes to an improved route in the preparation of *N*-(1-phenylethyl)anilines, it offers versatility to the route, and it enables the up-scaling of the process.

This optimized synthetic procedure is suitable for industrial production because it allows the production of *N*-(1-phenylethyl)anilines in increased yields, as it will be demonstrated in the illustrated examples hereinafter.

In a particular embodiment, this optimized synthetic procedure carries the additional advantage that it can be performed in a one-pot procedure, as it is described in the experimental part.

### Summary of the invention

One embodiment of the present invention provides a process for the preparation of a compound of formula (X), and pharmaceutically acceptable salts and solvates thereof, 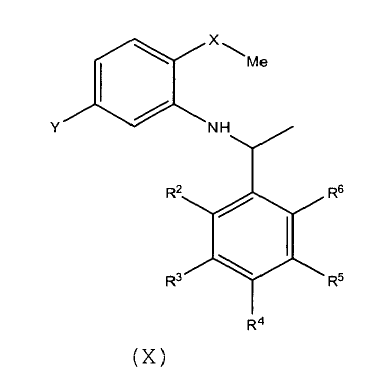 said process comprising the reaction of a compound of formula (IX) with a methyl nucleophile in an organic solvent at a temperature between -100 and 50°C; 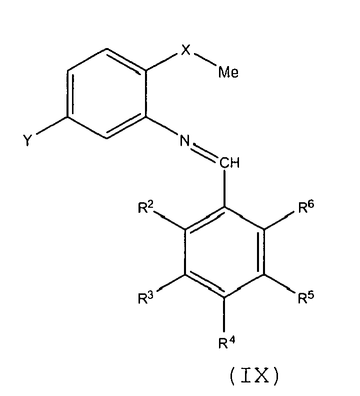 wherein in each of the compounds of formula (IX) and (X), independently,
- **Y**: is halo or 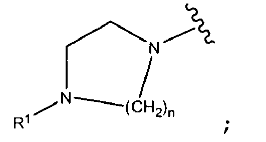
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂- or -S-;
- **R², R³, R⁴, R⁵, and R⁶**: are, each independently, hydrogen;halo; C₁₋₃alkoxy; or two adjacent substituents of the group of R², R³, R⁴, R⁵, and R⁶, together with the two carbon atoms of the benzene ring to which they are attached to, form the ring 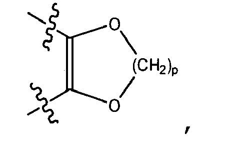 wherein **p** is 2, 3, 4, 5, or 6; and
- **R⁷**: is C₁₋₆alkyl or amino.

Another embodiment of the present invention provides a process for the preparation of a compound of formula (IX), as defined in the proceeding paragraph, said process comprising the reaction of a compound of formula (VII) with a compound of formula (VIII) under dehydrating conditions, optionally using a drying agent, and optionally in the presence of a catalyst, 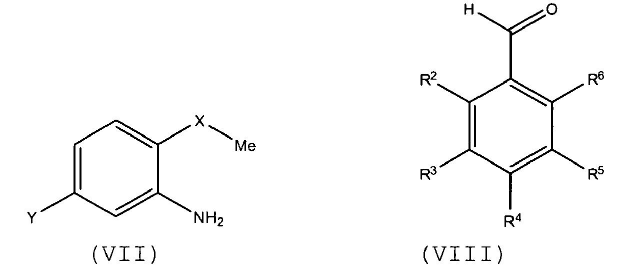 wherein
**Y, R¹, n, X, R², R³, R⁴, R⁵, and R⁶** are as defined above.

Another embodiment of the present invention provides a combination of the proceeding processes in a one-pot procedure.

Further embodiments of the present invention are directed to compounds *per se*. One of these embodiments provides those compounds of formula (IX), wherein
- **Y**: is halo, or 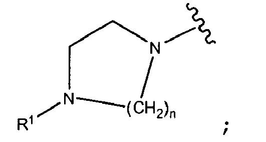
- **R¹**: is hydrogen, C₁₋₆alkyl optionally substituted with C₃₋₇cycloalkyl, phenyl optionally substituted with C₁₋₆alkyl or C₁₋₆alkoxy, -C(=O)-R⁷, or a protecting group;
- n: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent R², R³, R⁴, R⁵, and R⁶ together with the two carbon atoms of the benzene ring to which they are attached form the ring 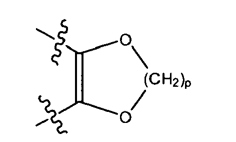 wherein **p** is 2, 3, 4, 5, or 6; and
- **R⁷**: is C₁₋₆alkyl or amino.

Another of these embodiments provides compounds of formula (X) *per se*, wherein
- **Y**: is halo;
- **X**: is -S-;
- **R², R³, R⁴, R⁵, and R⁶**: are, each independently, hydrogen; halo; C₁₋₃alkoxy; or two adjacent R², R³, R⁴, R⁵, and R⁶ together with the two carbon atoms of the benzene ring to which they are attached form the ring 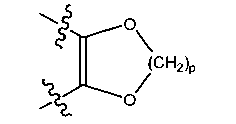
wherein **p** is 2, 3, 4, 5, or 6.

Another of these embodiments provides compounds of formula (VI) *per se*, 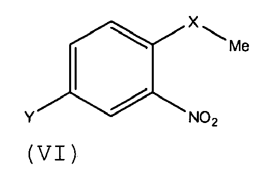 wherein
- **Y**: is 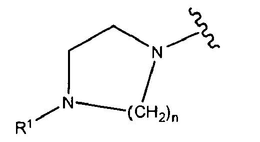
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂-; and
- **R⁷**: is C₁₋₆alkyl or amino.

Additional embodiments of the present invention include the use of compounds encompassed by the formulae (I), (II), (III), (IV), (V), (VI), (VII), (VIII), (IX), (X), (Xa), (Xb), and (Xc), as intermediates in the preparation of serotonin 5-HT6 antagonists, particularly those compounds of formula (IX), (X), and (VI).

### Disclosure of the invention

As used hereinbefore or hereinafter the term halo is generic to fluoro, chloro, bromo, and iodo. Similarly, the term halide is generic to fluorine, chlorine, bromine, and iodine.

The term C₁₋₃alkyl as a group or part of a group defines straight or branched chain saturated hydrocarbon radicals having from 1 to 3 carbon atoms such as methyl, ethyl, propyl, 1-methylethyl; C₁₋₆alkyl as a group or part of a group defines straight or branched chain saturated hydrocarbon radicals having from 1 to 6 carbon atoms such as the group defined for C₁₋₃alkyl, and n-butyl, isobutyl, sec-butyl, tert-butyl, pentyl, hexyl, 2-methylbutyl, and the like.

The term C₁₋₃alkoxy means C₁₋₃alkyloxy or a C₁₋₃alkyl ether radical, wherein the term C₁₋₃alkyl is as defined above. Examples of suitable C₁₋₃alkyl ether radicals include methoxy, ethoxy, n-propoxy, isopropoxy, and the like. C₁₋₆alkoxy means C₁₋₆alkyloxy or a C₁₋₆alkyl ether radical, wherein the term C₁₋₆alkyl is as defined above. Examples of suitable C₁₋₆alkyl ether radicals include those defined for C₁₋₃alkoxy, and n-butoxy, isobutoxy, sec-butoxy, tert-butoxy, pentoxy, hexoxy, 2-methylbutoxy, and the like.

The term C₃₋₇cycloalkyl is generic to cyclopropyl, cyclobutyl, cyclopentyl, cyclohexyl and cycloheptyl.

The term "protecting group" in R¹ refers to one or more selectively removable substituents on the amino group of the ring, commonly employed to block or protect the amino functionality against undesirable side reactions during synthetic procedures and includes all conventional amino protecting groups. Examples of amino-protecting groups include the urethane blocking groups, such as t-butoxycarbonyl (Boc), 2-(4-biphenylyl)propyl(2)oxycarbonyl (Bpoc), 2-phenylpropyl(2) oxycarbonyl (Poc), 2-(4-xenyl)isopropoxycarbonyl, isopropoxycarbonyl, 1, 1-diphenyletbyl(1)-oxycarbonyl, 1,1-diphenylpropyl(1)oxycarbonyl, 2-(3,5-dimethoxyphenyl)propyl(2)-oxycarbonyl (Ddz), 2-(p-5-toluyl)propyl(2) oxycarbonyl, 1-methylcyclopentanyloxycarbonyl, cyclohexanyloxycarbonyl, 1-methylcyclohexanyloxycarbonyl, 2-methylcyclohexanyloxycarbonyl, ethoxycarbonyl, 2-(4-toluylsulfonyl)ethoxycarbonyl, 2-(methylsulfonyl)-ethoxycarbonyl, 2-(triphenylphosphino)-ethoxycarbonyl, 9-fluoroenylmethoxycarbonyl (Fmoc), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, tribromoethoxycarbonyl, 2-ethynyl(2) propoxycarbonyl, cyclopropylmethoxycarbonyl, isobornyloxycarbonyl, 1-piperidyloxycarbonyl, benzyloxycarbonyl (Z or Cbz), 4-phenylbenzyloxycarbonyl, 2-methylbenzyloxy-carbonyl, α-2,4,5,-tetramethylbenzyloxycarbonyl (Tmz), 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, dichlorobenzyloxycarbonyl, 4-bromobenzyloxycarbonyl, ortho-bromobenzyloxycarbonyl, 3-bromobenzyloxycarbonyl, 4-nitrobenzyloxycarbonyl, 4-cyanobenzyloxycarbonyl, 4-(decyloxy) benzyloxycarbonyl, and the like; the benzoylmethylsulfonyl group, dithiasuccinoyl (Dts) group, the 2-(nitro)phenylsulfenyl group (Nps), the diphenylphosphine oxide group, and the like. The species of amino protecting group employed is usually not critical so long as the derivatized amino group is stable to the conditions of the subsequent reactions and can be removed at the appropriate point without disrupting the remainder of the compound.

It should be noted that the radical positions on any molecular moiety used in the definitions may be anywhere on such moiety as long as it is chemically stable.

Radicals used in the definitions of the variables include all possible isomers unless otherwise indicated. For instance pentyl includes 1-pentyl, 2-pentyl and 3-pentyl.

When any variable occurs more than one time in any constituent, each definition is independent.

The term "salt" as mentioned herein is meant to comprise any stable salts, which the compounds of the present invention, in particular those compounds of formula (X), (Xa), (Xb), and (Xc) are able to form. Preferred are the pharmaceutically acceptable salts, which are the non-toxic salt forms. Salts that are not pharmaceutically acceptable are also embraced in the scope of the present invention, when they refer to intermediates that are useful in the preparation of the final pharmaceutically active compounds.

The term "solvate" refers to those crystal forms of the compounds of formula (X), (Xa), (Xb), or (Xc) that contain either stoichiometric or non-stoichiometric amounts of solvent. Since water is a solvent, solvates also include hydrates. The term "pseudopolymorph" is synonym to solvate since it applies to polymorphic crystalline forms that have solvent molecules incorporated in their lattice structures. Examples of solvates are hydrates and alcoholates such as methanolates or ethanolates.

The present invention relates to a process for the preparation of a compound of formula (X), and pharmaceutically acceptable salts and solvates thereof, 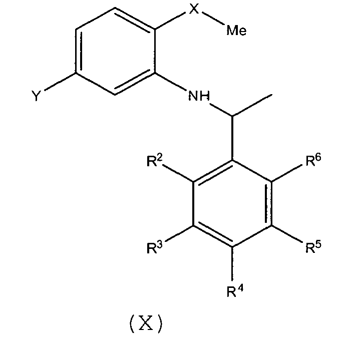 said process comprising the reaction of a compound of formula (IX) with a methyl nucleophile in an organic solvent at a temperature between -100 and 50°C; 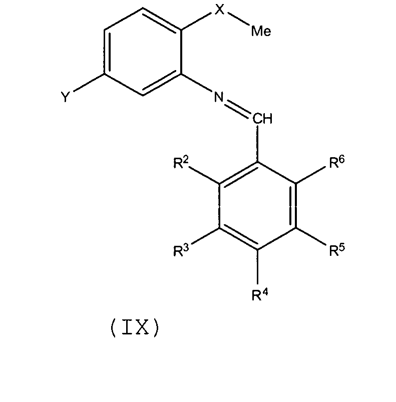 wherein in each of the compounds of formula (IX) and (X), independently,
- **Y**: is halo or 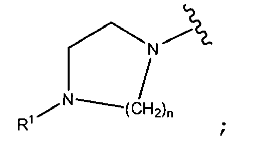
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- n: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 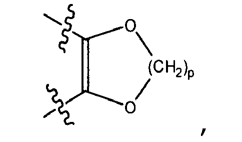
wherein **p** is 2, 3, 4, 5, or 6; and **R⁷** is C₁₋₆alkyl or amino.

In one embodiment, the present invention relates to process for the preparation of a compound of formula (IX), 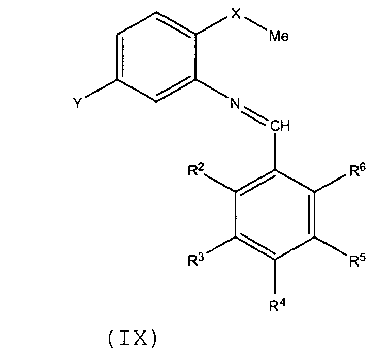 said process comprising the reaction of a compound of formula (VII) with a compound of formula (VIII) under dehydrating conditions, optionally using a drying agent, and optionally in the presence of a catalyst, 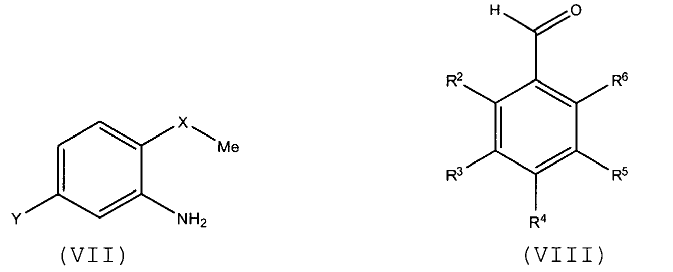 wherein
- **Y**: is halo or 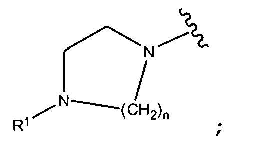
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- n: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 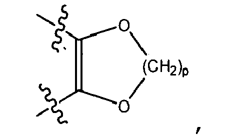 wherein **p** is 2, 3, 4, 5, or 6; and
- **R⁷**: is C₁₋₆alkyl or amino.

In another embodiment, the present invention relates to a process for the preparation of a compound of formula (X), 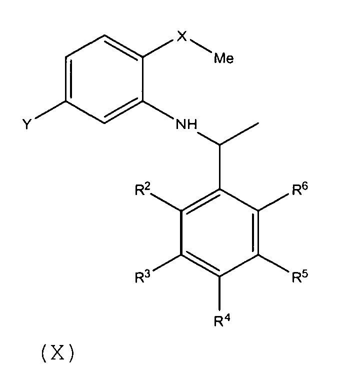 said process comprising the reaction of a compound of formula (VII) with a compound of formula (VIII) under dehydrating conditions, optionally using a drying agent, and optionally in the presence of a catalyst, 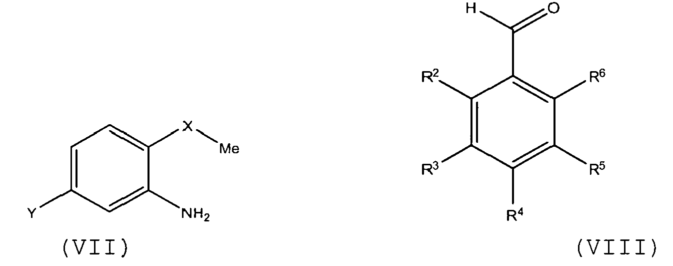 thereby obtaining compound of formula (IX) 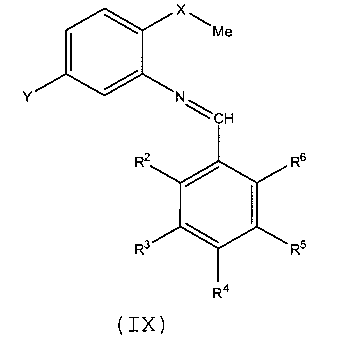 followed by the addition to the reaction mixture of a methyl nucleophile in an organic solvent at a temperature between -100 and 50°C;
wherein in compounds of formula (VII), (VIII), (IX), and (X)
- **Y**: is halo or 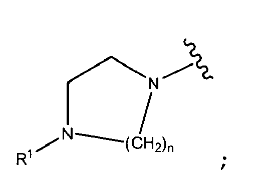
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 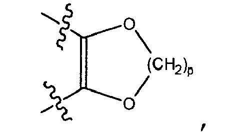 wherein **p** is 2, 3, 4, 5, or 6; and
- **R⁷**: is C₁₋₆alkyl or amino.

In another embodiment, the present invention relates to a process for the preparation of a compound of formula (X), and pharmaceutically acceptable salts and solvates thereof, 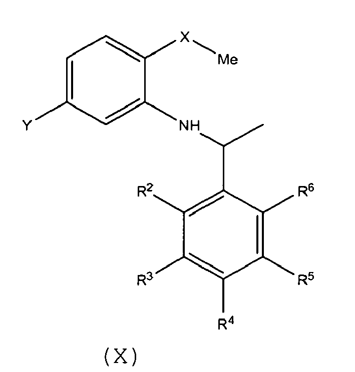 said process comprising the reaction of a compound of formula (IX) with a methyl nucleophile in an organic solvent at a temperature between -100 and 50°C; 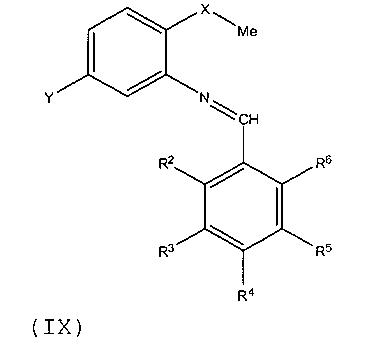 wherein compound of formula (IX) is obtained by reacting a compound of formula (VII) with a compound of formula (VIII) under dehydrating conditions, optionally using a drying agent, and optionally in the presence of a catalyst; 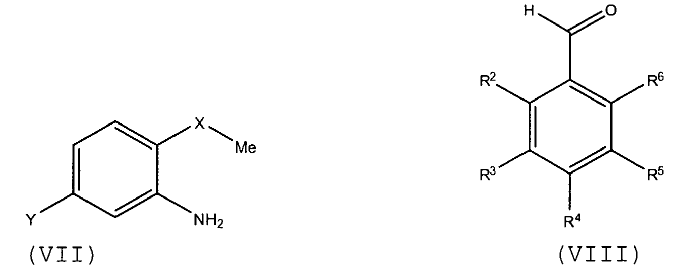 wherein in each of the compounds of formula (VII), (VIII), (IX), and (X), independently,
- **Y**: is halo or 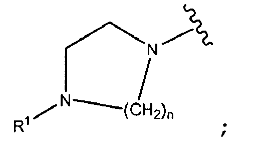
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 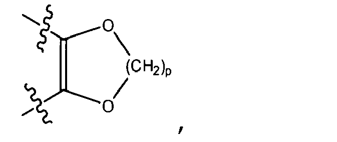 wherein **p** is 2, 3, 4, 5, or 6; and
- **R⁷**: is C₁₋₆alkyl or amino.

For ease of description and interpretation, the processes described above can be represented by the following scheme 1.

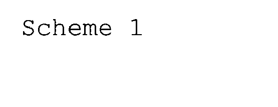

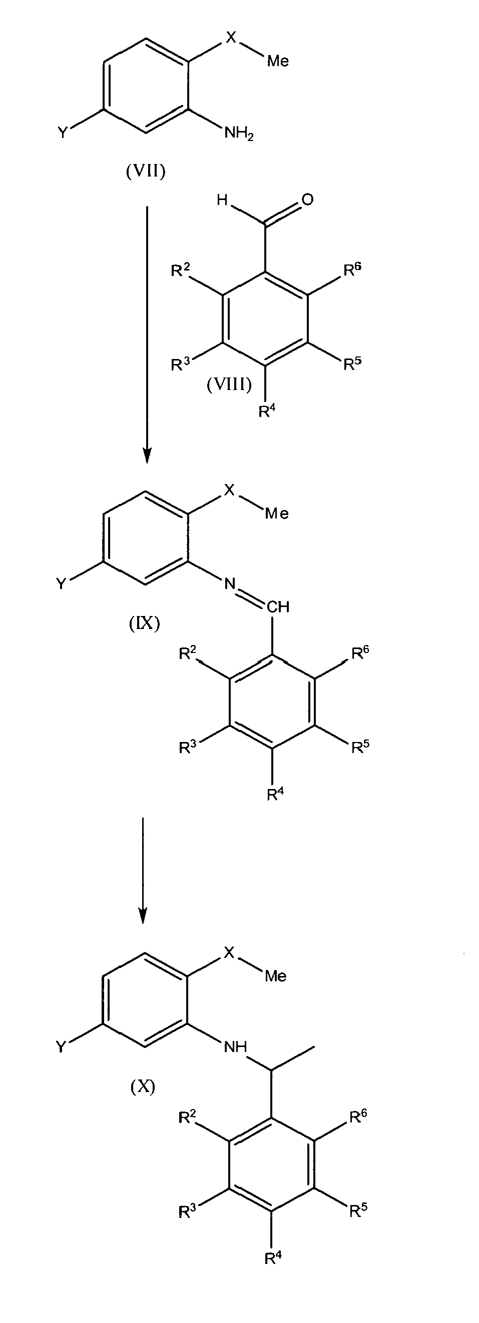

### Step (VII) + (VIII) → (IX)

The reaction of compound of formula (VII) with compound of formula (VIII) resulting in compound of formula (IX) is carried out under dehydrating conditions, optionally using a drying agent, and optionally in the presence of a catalyst.

In one embodiment of the present invention, the dehydrating conditions employed in the reaction of compound of formula (VII) with compound of formula (VIII) are the azeotropic removal of water from an organic solvent, or by using molecular sieves, amongst other. The organic solvent may be selected from benzene, toluene, tetrahydrofuran, xylene, and dichloromethane. Preferably, the organic solvent is toluene or tetrahydrofuran.

In one embodiment of the present invention, the reaction of compound of formula (VII) with compound of formula (VIII) may be carried out in the presence of a drying agent. Suitable drying agents can be selected inter alia from anhydrous MgSO₄, anhydrous NaSO₄, and molecular sieves. Preferably, the drying agent is selected from anhydrous MgSO₄, anhydrous NaSO₄, and molecular sieves.

In one embodiment of the present invention, the reaction of compound of formula (VII) with compound of formula (VIII) may be carried out without the presence of a drying agent.

In one embodiment of the present invention, the reaction of compound of formula (VII) with compound of formula (VIII) may be carried out in the presence of a catalyst. Suitable catalysts for this reaction may be selected from camphorsulphonic acid, sulfuric acid, p-toluenesulphonic acid, BF₃·Et₂O, SiO₂, TiCl₄, SnCl₂, ZnCl₂, Bi(OTf)₃, and AlCl₃. Preferably, the catalyst is selected from camphorsulphonic acid, SiO₂, TiCl₄, and p-toluenesulphonic acid.

In one embodiment of the present invention, the reaction of compound of formula (VII) with compound of formula (VIII) may be carried out without the presence of a catalyst.

In a preferred embodiment of the present invention, the reaction of compound of formula (VII) with compound of formula (VIII) is carried out under a combination of azeotropic distillation and a drying agent. In a more preferred embodiment, the reaction of compound of formula (VII) with compound of formula (VIII) is carried out under azeotropic removal of water with toluene as an organic solvent and employing molecular sieves as drying agent.

In a preferred reaction of compound of formula (VII) with compound of formula (VIII), under any one of the conditions mentioned in the proceeding paragraphs, Y is halo, and X is -S- in compound of formula (VII). These restrictions in compound of formula (VII) result in an increased reaction yield when synthesizing compound of formula (IX).

### Step (IX) → (X)

The conversion of a compound of formula (IX) into compound of formula (X) comprises the reaction of a compound of formula (IX) with a methyl nucleophile in an organic solvent at a temperature between -100 and 50°C.

In one embodiment of the present invention, the methyl nucleophile employed in the conversion of compound of formula (IX) into compound of formula (X) may be selected *inter alia* from a methyl magnesium halide and methyl lithium.

In one embodiment of the present invention, the organic solvent employed during the nucleophilic addition may be selected from tetrahydrofuran (THF), heptanes, dioxane, and toluene, amongst other. Preferably, the organic solvent of choice for the conversion of compound of formula (IX) into compound of formula (X) is toluene or THF, more preferably toluene.

In one embodiment, when one or more of the following restrictions apply to compound of formula (IX):
- **Y** is halo;
- **X** is -S-;
   the nucleophilic addition onto compound of formula (IX) is preferably carried out at a temperature between -100 and -40 °C, more preferably at a temperature between -85 and -70 °C.

In one embodiment, when one or more of the following restrictions apply to compound of formula (IX):
- **Y** is 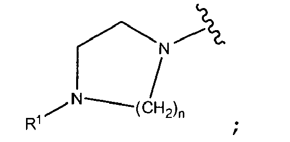
- **R¹** is hydrogen, C₁₋₆alkyl optionally substituted with C₃₋₇cycloalkyl, phenyl optionally substituted with C₁₋₆alkyl or C₁₋₆alkoxy, -C(=O)-R⁷, or a protecting group;
- **n** represents zero, 1, 2, 3, or 4; and
- **X** is -SO₂-;
   the nucleophilic addition onto compound of formula (IX) is preferably carried out at a temperature between -40 and -10 °C, more preferably at a temperature between -25 and -20 °C.

In a preferred embodiment of the present invention, the formation compound of formula (IX) and the addition of a methyl nucleophile to produce compound of formula (X) is carried out in a one-pot procedure. This one-pot procedure allows the simplification and economisation of the process, since it avoids the separation and purification of compound of formula (IX). As such, after reacting compound of formula (VII) with compound of formula (VIII) under dehydrating conditions, optionally using a drying agent, and optionally in the presence of a catalyst, the resulting reaction mixture -comprising compound of formula (IX)- is directly cooled at a temperature between -100 and 50°C and the methyl nucleophile is added in a suitable organic solvent.

In those embodiments of the present invention, wherein in the obtained compound of formula (X),
- **Y** is halo;
- **X** is -S-;
   such compound of formula (X) can be further reacted to introduce the desired substituents -the optionally substituted heterocyclic ring and the sulfonyl moiety, respectively- and obtain the final product, i.e. compound of formula (Xc), and the salts and solvates thereof.

As such, the reactions that compound of formula (X) may undergo in the preparation of the compound of formula (Xc) can be represented by the following scheme 2.

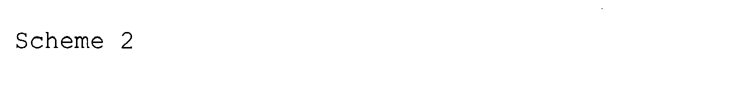

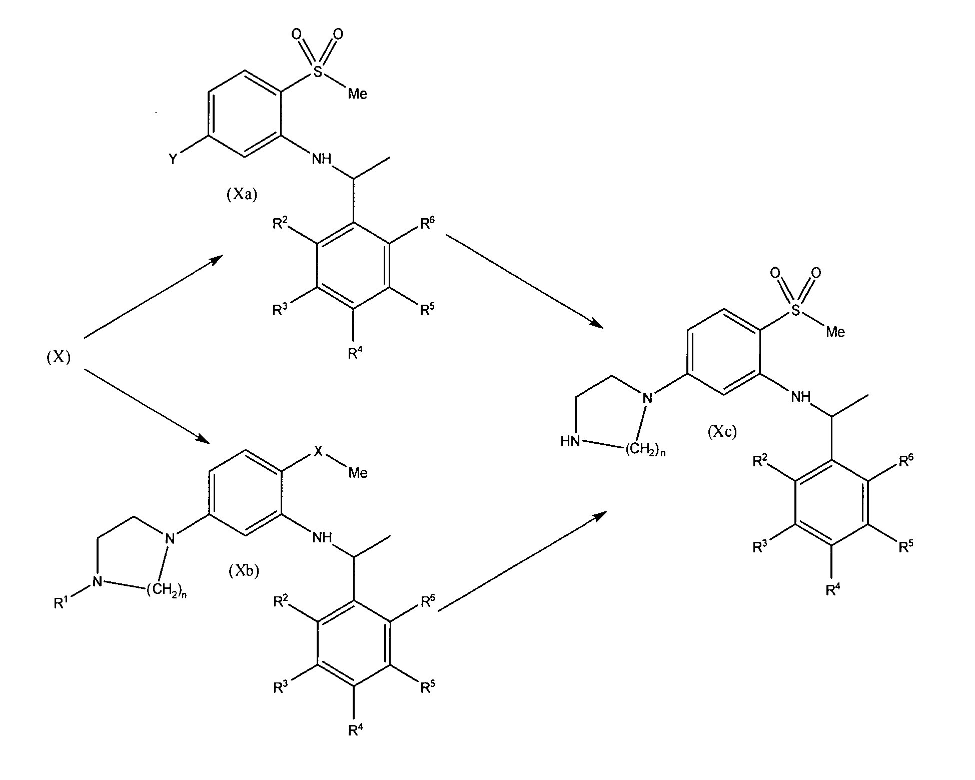

As such, in one embodiment, the present invention relates to a process for the preparation of a compound of formula (Xa), and pharmaceutically acceptable salts and solvates thereof, 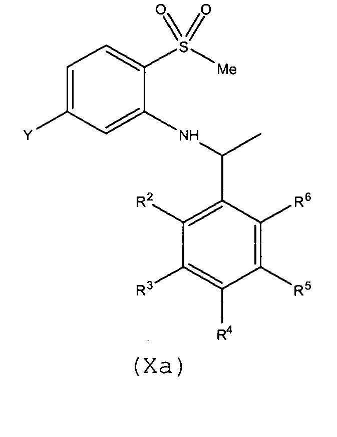 said process comprising the reaction of a compound of formula (IX) with a methyl nucleophile in an organic solvent at a temperature between -100 and 50°C; 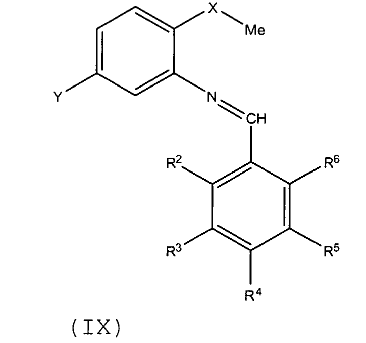 resulting in a compound of formula (X), which is further reacted with an oxidant in a solvent selected from water, one or more organic solvents, and mixtures thereof, thereby obtaining compound of formula (Xa), and pharmaceutically acceptable salts and solvates thereof, 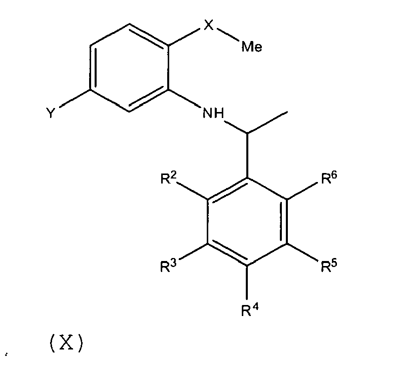 wherein in each of the compounds of formula (IX), (X), and (Xa), independently,
- **Y**: is halo or 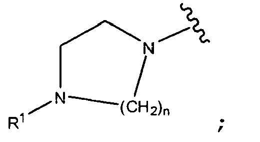
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 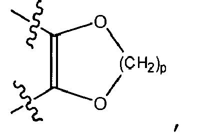 wherein **p** is 2, 3, 4, 5, or 6; and
- **R⁷**: is C₁₋₆alkyl or amino.

In another embodiment, the present invention relates to a process for the preparation of a compound of formula (Xb) or (Xc) and pharmaceutically acceptable salts and solvates thereof, 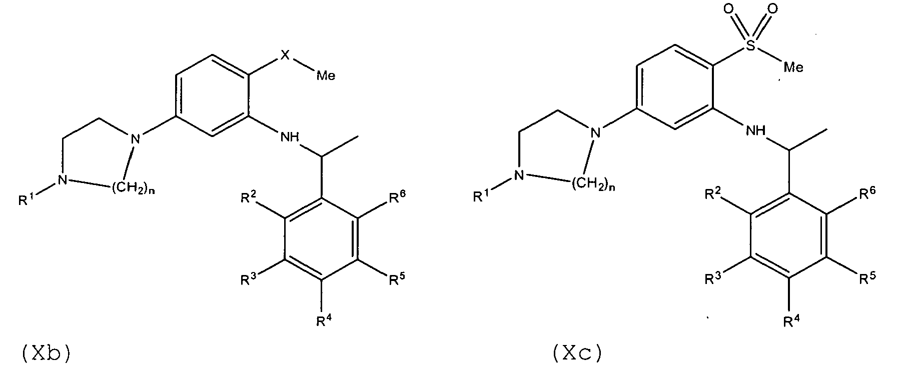 said process comprising
- the reaction of a compound of formula (IX) with a methyl nucleophile in an organic solvent at a temperature between -100 and 50°C; 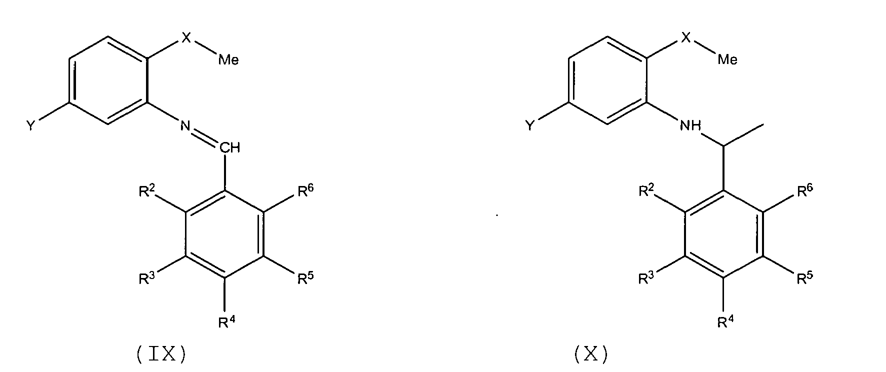 resulting in a compound of formula (X); or
- the additional reaction of compound of formula (X) with an oxidant in a solvent selected from water, one or more organic solvents, and mixtures thereof, thereby obtaining compound of formula (Xa); 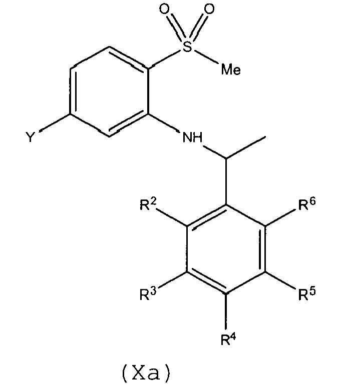 which compound of formula (X) or (Xa) is further reacted with a compound of formula (IV) in an organic solvent, optionally in the presence of a base, at a temperature between 20 and 150 °C, 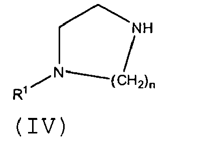 thereby obtaining compounds of formula (Xb) and (Xc), respectively; 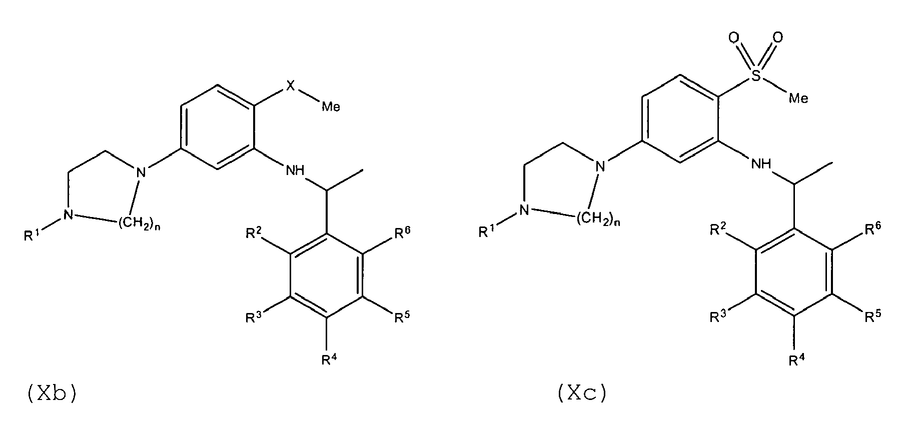
wherein in each of the compounds of formula (IV), (IX), (X), (Xa), (Xb), and (Xc), independently, and where applicable,
- **Y**: is halo;
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 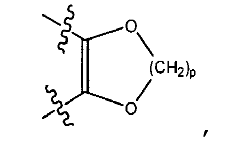 wherein **p** is 2, 3, 4, 5, or 6; and
- **R⁷**: is C₁₋₆alkyl or amino.

In another embodiment, the present invention relates to a process for the preparation of a compound of formula (Xc) and pharmaceutically acceptable salts and solvates thereof, 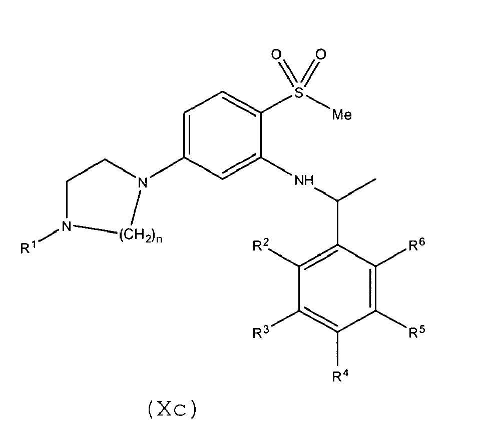 said process comprising the reaction of a compound of formula (IX) with a methyl nucleophile in an organic solvent at a temperature between -100 and 50°C; 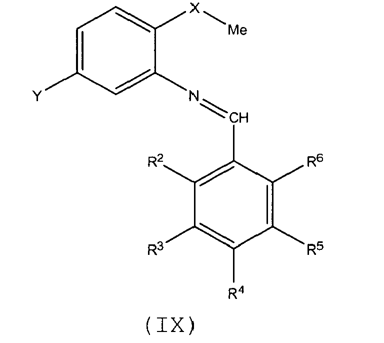 resulting in a compound of formula (X), 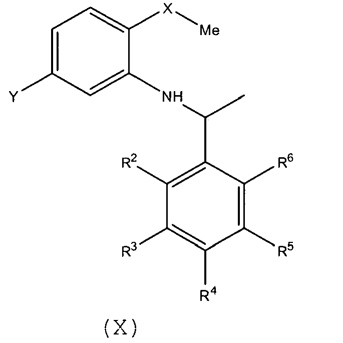 which compound of formula (X) is further reacted with a compound of formula (IV) in an organic solvent, optionally in the presence of a base, at a temperature between 20 and 150 °C, 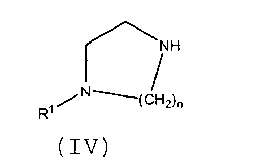 thereby obtaining compound of formula (Xb), 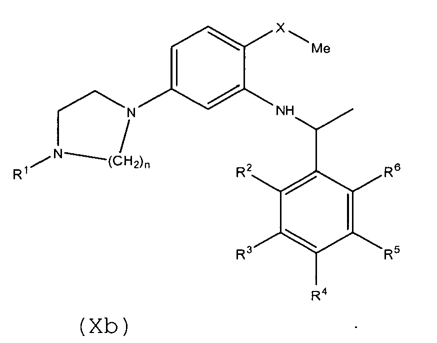 which compound of formula (Xb) is further reacted with an oxidant in a solvent selected from water, one or more organic solvents, and mixtures thereof, thereby obtaining compound of formula (Xc), and pharmaceutically acceptable salts and solvates thereof, 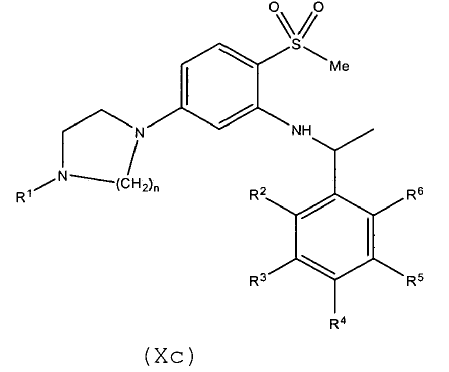 wherein in each of the compounds of formula (IV), (IX), (X), (Xb), and (Xc), independently, and where applicable,
- **Y**: is halo;
- **R¹ R³, R⁴, R⁵, and R⁶**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -S-;
- **R²,**: are, each independently, hydrogen; halo; C₁₋₃alkoxy; or two adjacent substituents of the group of R², R³, R⁴, R⁵, and R⁵, together with the two carbon atoms of the benzene ring to which they are attached to, form the ring 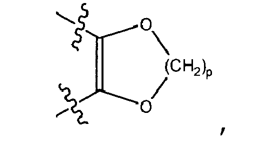 wherein **p** is 2, 3, 4, 5, or 6; and
- **R⁷**: is C₁₋₆alkyl or amino.

### Step (X) → (Xa)

The production of compound of formula (Xa), pharmaceutically acceptable salts and solvates thereof, is performed by reacting compound of formula (X) with an oxidant in a solvent selected from water, one or more organic solvents, and mixtures thereof.

In one embodiment of the present invention, the oxidant may be selected from peracetic acid, trifluoroperacetic acid, oxone® (potassium peroxymonosulfate), H₂O₂, 3-chloroperoxy-benzoic acid *(m*-CPBA), (±)-*trans-*2-(phenylsulfonyl)-3-phenyloxyaziridine, oxaziridine, amongst other. Preferably, the oxidant is selected from m-CPBA and oxone®, more preferably the oxidant is oxone®, which is comprised in an organic solvent such as water, mixtures of water with organic solvents, or mixtures of organic solvents including but not limited to dichloromethane, methanol, THF, dimethylether, or methyl tert-butyl ether (MTBE). Also preferably, the solvent comprising the oxidant is a mixture of water and organic solvents. More preferably, the oxidant is dissolved in a mixture of THF/MeOH/water at a temperature between -20 and 60 °C, preferably at a temperature between 0 and 40 °C, and more preferably at a temperature between 15 and 25 °C.

### Steps (X) → (Xb) and (Xa) → (Xc)

The production of compound of formulae (Xb) or (Xc), pharmaceutically acceptable salts and solvates thereof, is performed by reacting compound of formula (X) or (Xa), respectively, with a compound of formula (IV) in an organic solvent, optionally in the presence of a base, at a temperature between 20 and 150 °C, 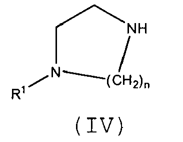 thereby obtaining compound of formula (Xb) or (Xc), respectively, and the pharmaceutically acceptable salts and solvates thereof.

The organic solvent that can be used during the conversion of compounds of formula (X) or (Xa) into compounds of formula (Xb) or (Xc), respectively, may be selected from toluene, N-methylpyrrolidinone (NMP), dimethylformamide (DMF), dimethoxyethane (DME), dimethylacetamide (DMA), or dimethyl sulfoxide (DMSO), preferably DMSO, DMA, and NMP, more preferably DMA.

The base that may be optionally added to the reaction mixture for the conversion of compounds of formula (X) or (Xa) into compounds of formula (Xb) or (Xc), respectively, may be selected from potassium carbonate, cesium carbonate, and triethylamine. In one preferred embodiment, the conversion of compounds of formula (X) or (Xa) into compounds of formula (Xb) or (Xc) is carried out in the absence of a base.

The conversion of compounds of formula (X) or (Xa) into compounds of formula (Xb) or (Xc), respectively, may be carried out at a temperature ranging between 20 and 150 °C, preferably at a temperature between 80 and 150 °C, more preferably at a temperature between 120 and 140 °C, even more preferably at a temperature between 125 and 130 °C.

In an alternative embodiment, the coupling of compounds of formula (X) or (Xa) with compound of formula (IV) in order to obtain compounds of formula (Xb) or (Xc), respectively, may be performed under organometallic coupling conditions in a suitable organic solvent. These organometallic coupling conditions include palladium-catalyzed reactions, Buchwald or Ullmann conditions, amongst other.

### Step (Xb) → (Xc)

The production of compound of formula (Xc), pharmaceutically acceptable salts and solvates thereof, is performed by reacting compound of formula (Xb) with an oxidant in a solvent selected from water, one or more organic solvents, and mixtures thereof.

In one embodiment of the present invention, the oxidant may be selected from peracetic acid, trifluoroperacetic acid, oxone®, H₂O₂, *m*-CPBA, (±)-*trans*-2-(phenylsulfonyl)-3-phenyloxyaziridine, oxaziridine, amongst other. Preferably, the oxidant is selected from m-CPBA and oxone®, more preferably the oxidant is oxone®, which is comprised in an organic solvent such as water, mixtures of water with organic solvents, or mixtures of organic solvents including but not limited to dichloromethane, methanol, THF, dimethylether, or MTBE. Also preferably, the solvent comprising the oxidant is a mixture of water and organic solvents. More preferably, the oxidant is dissolved in a mixture of THF/methanol/water at a temperature between -20 and 60 °C, preferably at a temperature between 0 and 40 °C, and more preferably at a temperature between 15 and 25 °C.

The compounds of formula (X), (Xa), (Xb), and (Xc) have at least one center of chirality and exist as stereochemically isomeric forms. The term "stereochemically isomeric forms" as used herein defines all the possible compounds made up of the same atoms bound by the same sequence of bonds but having different three-dimensional structures which are not interchangeable, which the compounds of formula (X), (Xa), (Xb), or (Xc) may possess.

With reference to the instances where (R) or (S) is used to designate the absolute configuration of a chiral atom within a substituent, the designation is done taking into consideration the whole compound and not the substituent in isolation.

Unless otherwise mentioned or indicated, the chemical designation of a compound encompasses the mixture of all possible stereochemically isomeric forms, which said compound may possess. Said mixture may contain all diastereomers and/or enantiomers of the basic molecular structure of said compound. All stereochemically isomeric forms of the compounds of the present invention both in pure form or mixed with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyl-tartaric acid, ditoluoyltartaric acid and camphosulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound is synthesized by stereospecific methods of preparation. These methods will advantageously employ enantiomerically pure starting materials.

The diastereomeric racemates of the compounds of formula (I) can be obtained separately by conventional methods. Appropriate physical separation methods that may advantageously be employed are, for example, selective crystallization and chromatography, e.g. column chromatography.

For some of the compounds of formula (X), (Xa), (Xb), or (Xc), their salts, solvates, and the intermediates used in the preparation thereof, the absolute stereochemical configuration was not experimentally determined. A person skilled in the art is able to determine the absolute configuration of such compounds using art-known methods such as, for example, X-ray diffraction.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

In scheme 3, there is represented a synthetic path leading to compound of formula (VII).

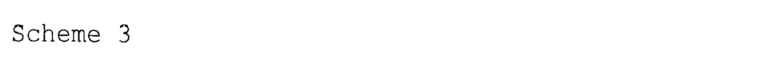

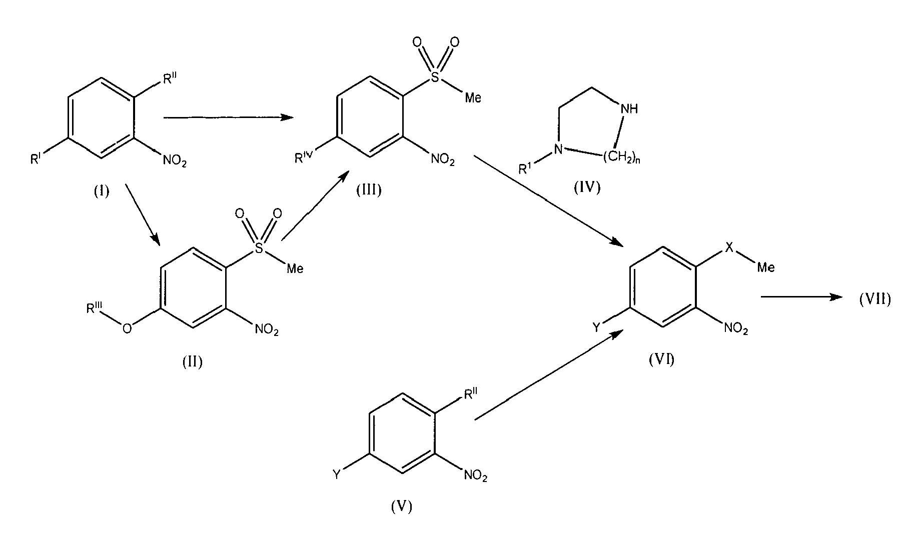

### Step (VI) → (VII)

Compound of formula (VII), a useful intermediate in the preparation of compounds of formulae (IX), (X), (Xa), (Xb), and (Xc), may be prepared by treating a compound of formula (VI) with a reducing agent in an organic solvent at a temperature between -20 and 80° C, 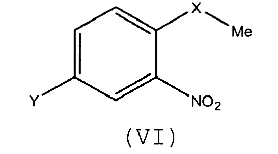 wherein
- **Y**: is halo or 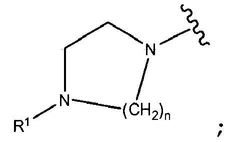 and
- **X**: is -SO₂- or -S-.

The reduction of the nitro group is performed by treating a compound of formula (VI) with a reducing agent such as a H₂ source and a metal catalyst such as Pd, Pt, SnCl₂, or Fe in acidic media. Preferably the reduction is carried out using catalytic hydrogenation with an heterogeneous catalyst, more preferably using Pd on carbon as a catalyst under H₂ atmosphere. Also preferably, the reduction is carried out using SnCl₂ with aqueous hydrochloric acid.

The reduction of the nitro group is performed in an organic solvent selected from ethyl acetate, isopropyl acetate, THF, MTBE, and alcohols such as methanol or ethanol; preferably the organic solvent employed in the reduction of the nitro group is methanol or THF; more preferably THF. Alternatively, the reduction of the nitro group is performed in an organic solvent selected from C₁₋₄alcohols such as methanol, ethanol, and isopropanol, preferably methanol or ethanol, more preferably ethanol.

The reaction temperature during the reduction of the nitro group is set at between -20 and 80 °C, preferably at a temperature ranging between 0 and 40 °C, and more preferably at a temperature between 15 and 25 °C. Alternatively, the reduction of the nitro group is performed at a temperature between 20 and 80 °C, preferably at a temperature between 40 and 60 °C, and more preferably at a temperature between 50 and 55 °C.

### Step (III) + (IV) → (VI)

In one embodiment of the present invention, there is provided a process for obtaining those compounds of formula (VI), wherein Y is 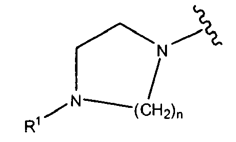 and X is -SO₂-, which process comprises the reaction of a compound of formula (III) with a compound of formula (IV) in an organic solvent, optionally in the presence of a base, at a temperature between 20 and 150 °C; 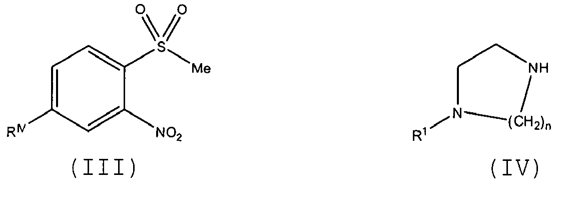 wherein
- **R^{IV}**: is halo, or a sulfonate selected from nonaflate, tresylate, nosylate, mesylate, tosylate, brosylate, and triflate;
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- n: represents zero, 1, 2, 3, or 4; and
- **R⁷**: is C₁₋₆alkyl or amino.

The organic solvent employed during the coupling of compound of formula (III) with compound of formula (IV) may be selected from toluene, NMP, DMF, DME, DMA, and DMSO, preferably the organic solvent is selected from DMSO, DMA, and NMP; more preferably the organic solvent is NMP.

In one embodiment of the present invention, the coupling of compound of formula (III) with compound of formula (IV) is conducted in the presence of a base selected from potassium carbonate, cesium carbonate, and triethylamine. In an alternative embodiment of the present invention, the coupling of compound of formula (III) with compound of formula (IV) is conducted in the absence of a base.

The temperature employed during the coupling of compound of formula (III) with compound of formula (IV) is set at between 20 and 150 °C, preferably at 20 to 120 °C, more preferably between 40 and 100 °C, and more preferably between 60 and 80 °C.

### Step (II) → (III)

In one embodiment of the present invention, there is provided a process for obtaining those compounds of formula (III), wherein R^{IV} is a sulfonate, which process comprises deprotecting compound of formula (II) and reacting the resultant hydroxyl group with a sulfonylating agent, in an organic solvent, in the presence of a base, 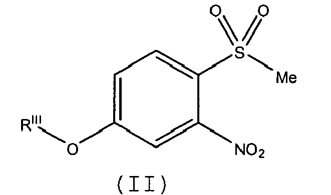 wherein
**R^{III}** is C₁₋₆alkyl, benzyl, silyl, p-methoxybenzyl, or - C(=O)-R^{V}; and
**R^{V}** is hydrogen, C₁₋₆alkyl, phenyl, *o*-tolyl, *m*-tolyl, *p-*tolyl, or *p*-nitrophenyl.

As such, the sulfonate moiety of R^{IV} in compound of formula (III) may be selected from nonaflate, tresylate, nosylate, mesylate, tosylate, brosylate, and triflate, amongst other. Preferably the sulfonate moiety of R^{IV} in compound of formula (III) is triflate or tosylate, more preferably the sulfonate moiety is triflate.

Deprotection of the hydroxyl-protecting group in compound of formula (II) may be performed by several methods known in the art, such as by alkaline hydrolysis, under acid conditions, by hydrolysis in general, and by deprotection reaction of a silyl group, amongst other.

The deprotection reaction by alkaline hydrolysis may be carried out at temperature between 0 and 40° C in an organic solvent such as methanol, tetrahydrofuran, dioxane, or mixtures thereof, using an alkali metal such as sodium hydroxide, potassium hydroxide, and lithium hydroxide, an alkaline earth metal such as barium hydroxide or calcium hydroxide, a carbonate such as sodium carbonate or potassium carbonate.

The deprotection reaction under acid conditions may be carried out at temperature between 0 and 100° C, using an organic acid such as acetate, trifluoroacetic acid, and methanesulfonic acid, or an inorganic acid such as hydrochloric acid and sulfate. under the presence or absence of an organic solvent such as methylene chloride, chloroform, dioxane, ethyl acetate, anisole, methanol, ethanol, and isopropyl alcohol.

The deprotection reaction by hydrolysis in general may be carried out at the temperature between 0 and 200° C in a solvent such as ethers like tetrahydrofuran, dioxane, dimethoxyethane, and diethyl ether, alcohols like methanol and ethanol, benzenes like benzene and toluene, ketone like acetone and methyl ethyl ketone, nitriles like acetonitrile, amides like dimethyl formamide, water, ethyl acetate, acetate, or mixtures thereof, under the presence of a catalyst such as palladium-carbon, palladium black, hydroxide palladium, platinum oxide, and raney nickel, at atmospheric or pressurized hydrogen atmosphere, or formate ammonium.

The deprotection reaction of a silyl group may be carried out at temperature between 0 and 40° C in water or an organic solvent like tetrahydrofuran and acetonitrile, and mixtures thereof, using tetrabutylammoniumfluoride.

The sulfonylating agent that can react with the resultant hydroxyl group may be selected from N-phenyl-bis(trifluoromethanesulfonimide), methanesulfonyl chloride, trifluoromethanesulfonyl chloride, methanesulfonic anhydride, trifluoromethanesulfonic anhydride, benzenesulfonyl chloride, toluenesulfonyl chloride, benzenesulfonyl bromide, toluenesulfonyl bromide, preferably *N*-phenyl-bis(trifluoromethanesulfonimide),
trifluoromethanesulfonic anhydride, or toluenesulfonyl chloride, more preferably trifluoromethanesulfonic anhydride.

The organic solvent that can be employed during the sulfonylation reaction may be selected from halogenated hydrocarbons, aromatic hydrocarbons, and aliphatic hydrocarbons. Examples of suitable organic solvents include THF, toluene, dichloromethane, dioxane, preferably dichloromethane and THF, and more preferably dichloromethane.

Examples of a base that can be used during the sulfonylation reaction include, inter alia, amines such as triethylamine, tributylamine, diisopropylethylamine (DIPEA), pyridine, picoline, lutidine, 4-dimethylaminopyridine, diaza(1,3)bicyclo[5.4.0]undecane (DBU), 1,4-diazabicyclo[2.2.2]octane (DABCO), 1,5-diazabicyclo(4.3.0)non-5-ene (DBN). Preferable bases are DIPEA and triethylamine; a more preferable base is triethylamine.

The sulfonylation reaction is carried out in a temperature range usually between -100 and 50° C, preferably at a temperature between -90 and room temperature. Incidentally, the temperature may be warmed up during the reaction.

### Steps (I) → (III) and (I) → (II)

In one embodiment of the present invention, there is provided a process for obtaining a compound of formula (II), or those compounds of formula (III) wherein R^{IV} is halo. Such compounds may be obtained by reacting a compound of formula (I) with a sulphur nucleophile in an organic solvent, at a temperature between 50 and 150°C, 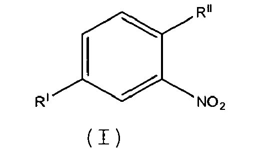 wherein
**R^{I}** is halo or -OR^{III};
**R^{II}** is halo;
**R^{III}** is C₁₋₆alkyl, benzyl, silyl, p-methoxybenzyl, or - C(=O)-R^{V}; and
**R^{V}** is hydrogen, C₁₋₆alkyl, phenyl, o-tolyl, *m*-tolyl, *p-*tolyl, or p-nitrophenyl.

The sulphur nucleophile may be selected from sulphides, hydrogensulphides, thiocarbonic acid, thiosulphonic acid, thiophosphoric acid, and thiourea. Examples of sulphur nucleophiles include thiolacetic acid, sodium thiolacetate, thiophenol, mercaptoacetic acid, sodium sulphite, and sodium methanesulfinate. A preferred sulphur nucleophile is sodium methanesulfinate.

The organic solvent employed in the reaction with the sulphur nucleophile above is selected from DMSO, DMF, and acetonitrile. Preferably, the organic solvent is DMF or DMSO, more preferably the organic solvent is DMSO.

The reaction with the sulphur nucleophile is carried out at a temperature between 50 and 150°C, preferably at a temperature between 50 and 100 °C, and more preferably at a temperature between 75 °C and 85 °C.

For those compounds of formula (I) where R^{I} is halo, those preferred are the ones where R^{I} is fluoro or chloro. More preferred are those compounds of formula (I) where R^{I} is fluoro.

Preferred compounds of formula (I) are also those where R^{II} is fluoro or chloro. More preferred are those compounds of formula (I) where R^{II} is fluoro.

Compounds of formula (I), wherein both R^{I} and R^{II} are halo, are for example:
- 1,4-difluoro-2-nitrobenzene [CAS: 364-74-9];
- 2-chloro-5-fluoronitrobenzene [CAS: 345-17-5];
- 1-Bromo-4-fluoro-2-nitrobenzene [CAS: 446-09-3];
- 4-fluoro-1-iodo-2-nitrobenzene [CAS: 364-77-2];
- 2,5-dichloro-1-nitrobenzene [CAS: 89-61-2];
- 5-chloro-2-fluoro-nitrobenzene [CAS: 345-18-6];
- 1-bromo-4-chloro-2-nitrobenzene [CAS: 41513-04-6];
- 1-bromo-4-fluoro-3-nitrobenzene [CAS: 364-73-8];
- 2,5-dichloro-1-nitrobenzene [CAS: 89-61-2]; and
- 2,5-dibromonitrobenzene [CAS: 3460-18-2];
   which are commercially available from Sigma Aldrich, Acros Organics, Alfa Aesar, Apollo Scientific Ltd, Fluorochem Limited, Fluka, ABCR, amongst other.

Compounds of formula (I), wherein R^{I} is methoxy and R^{II} is halo, are for example:
- 4-chloro-3-nitroanisole [CAS: 10298-80-3];
- 4-bromo-3-nitroanisole [CAS: 5344-78-5]; or
- 4-iodo-3-nitroanisole [CAS: 58755-70-7];
   which are commercially available from Sigma Aldrich, Acros Organics, Alfa Aesar, Apollo Scientific Ltd, Fluorochem Limited, Fluka, ABCR, amongst other.

### Step (V) → (VI)

In one embodiment of the present invention, there is provided a process for the preparation of those compounds of formula (VI) wherein X is -S- and Y is halo, said process comprising the reaction of compound of formula (V) with a methyl-bearing sulphur nucleophile, in a mixture of an organic solvent and water, at a temperature between -40 and 150 °C, 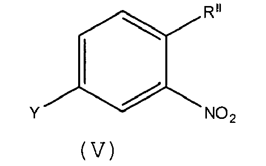 wherein
**Y** is halo; and
**R^{II}** is halo.

The introduction of the thiomethyl ether onto compound of formula (V) is carried out with a methyl-bearing sulphur nuclophile such as sodium thiomethoxide, potassium thiomethoxide, or another suitable salt.

The reaction is usually carried out in an organic solvent including ethers such as tetrahydrofuran, diethyl ether, and methyl t-butyl ether; acid amides such as N,N-dimethylformamide; nitrites such as acetonitrile; alcohols such as methanol and ethanol; dimethylsulfoxide; acetonitrile; NMP; mixtures thereof; and mixtures of the above organic solvents with water. A preferred organic solvent is DMF or a mixture of DMSO and water. A more preferred organic solvent is a DMF and water mixture.

The reaction temperature is usually in the range of -40 to 150 °C, preferably between -20 and 20 °C, and more preferably between 0 and 5 °C.

Compounds of formula (V), wherein both Y and R^{II} are halo, are for example:
- 1,4-difluoro-2-nitrobenzene [CAS: 364-74-9];
- 2-chloro-5-fluoronitrobenzene [CAS: 345-17-5];
- 1-Bromo-4-fluoro-2-nitrobenzene [CAS: 446-09-3];
- 4-fluoro-1-iodo-2-nitrobenzene [CAS: 364-77-2];
- 2,5-dichloro-1-nitrobenzene [CAS: 89-61-2];
- 5-chloro-2-fluoro-nitrobenzene [CAS: 345-18-6];
- 1-bromo-4-chloro-2-nitrobenzene [CAS: 41513-04-6];
- 1-bromo-4-fluoro-3-nitrobenzene [CAS: 364-73-8];
- 2,5-dichloro-1-nitrobenzene [CAS: 89-61-2];
- 2,5-dibromonitrobenzene [CAS: 3460-18-2];
   which are commercially available from Sigma Aldrich, Acros Organics, Alfa Aesar, Apollo Scientific Ltd, Fluorochem Limited, Fluka, ABCR, amongst other.

In the above-mentioned respective reactions, each of the obtained compounds, when necessary, can be collected from the reaction mixture according to the methods known in the art. For example, when insoluble materials are present, the desired compound can be obtained -after removing the insoluble materials by filtration- by removing the solvent, e.g. by removing the solvent under reduced pressure, and/or by adding water to the residue and extracting the mixture with a water-immiscible organic solvent such as ethyl acetate, etc. Optionally, the desired compound can be obtained after drying over anhydrous sodium sulfate, for instance, and further, if necessary, by purifying with any conventional method, such as recrystallization, column chromatography, or other techniques.

In one embodiment of the present invention, there is provided an additional process wherein compound of formula (X), (Xa), (Xb), or (Xc) is further reacted with an acid in an organic solvent, thereby obtaining a salt of compound of formula (X), (Xa), (Xb), or (Xc), respectively. As such, compounds of the formula (X), (Xa), (Xb), or (Xc) can be converted into a pharmaceutically acceptable salt by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propane-tricarboxylic, methanesulfonic, ethanesulfonic, benzenesulfonic, 4-methylbenzene-sulfonic, cyclohexanesulfamic, 2-hydroxybenzoic, 4-amino-2-hydroxybenzoic, and the like acids. The formation of the salt is suitably carried out in an inert solvent, preferably ethers such as diethyl ether, tetrahydrofuran, dimethoxyethane, diethoxyethane, dioxane, etc.; alcohols such as methanol, ethanol, propanol, isopropanol, butanol, etc.; halogenated hydrocarbons such as methylene chloride, chloroform, etc.; or water). After reaction of the base form with such appropriate acids, the solvent is removed, or else, precipitated crystals are collected by filtration to obtain the desired salt. Also, it can be directly separated as a salt from the reaction mixture in the final reaction step. Conversely the salt form can be converted by treatment with alkali into the free base form.

A preferred salt of compounds of the formula (X), (Xa), (Xb), or (Xc) is the hydrochloride salt thereof, which can be prepared by addition of hydrochloric acid to any one of compounds of formula (X), (Xa), (Xb), or (Xc), respectively. The hydrochloric acid may be applied as HCl gas, or dissolved in an organic solvent or aqueous solution. Preferably, the hydrochloric acid is presented in solution in an organic solvent selected from ethanol, isopropanol, dioxane, and diethyl ether; more preferably the organic solvent is dioxane or diethyl ether, even more preferably the organic solvent is diethyl ether. The reaction mixture is optionally dissolved in an organic solvent selected from ethyl acetate, isopropyl acetate, n-butyl acetate, tetrahydrofuran, diethyl ether; preferably in diethyl ether, tetrahydrofuran, or ethyl acetate; more preferably in ethyl acetate.

The reaction temperature during the formation of the salt is between -20 and 80 °C, preferably the temperature is between 0 and 40 °C and more preferably the temperature is between 15 and 25 °C.

The resulting solid may be optionally re-suspended in an organic solvent and concentrated to remove excess of the acid. This operation may be carried out as many times as necessary.

A further embodiment of the present invention, relates to a compound of formula (IX) *per se* 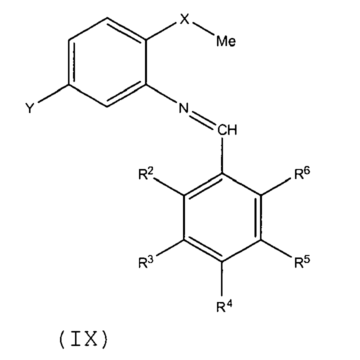 wherein
- **Y**: is halo, or 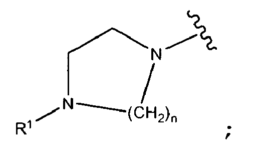
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **X**: is -SO₂- or -S-;
- **R², R³, R⁴, R⁵, and R⁶**: are, each independently, hydrogen; halo; C₁₋₃alkoxy; or two adjacent R², R³, R⁴, R⁵, and R⁶ together with the two carbon atoms of the benzene ring to which they are attached form the ring 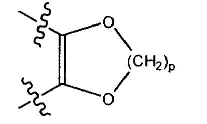 wherein **p** is 2, 3, 4, 5, or 6; and
- **R⁷**: is C₁₋₆alkyl or amino.

In compound of the formula (IX), there exists 2 geometric isomers (E,Z) due to the presence of an unsaturated carbon. The respective isomers can be separated by treating the corresponding racemic isomers or geometric isomer mixture by usual optical resolution methods such as fractional recrystallization method, optical resolution column chromatography method, or diastereomer method; or separation methods such as recrystallization method, column chromatography method, amongst other. For example, when optical isomers are to be separated, the racemic mixture of compound of formula (IX) is reacted with an optically active sulfonic acid compound, e.g. (S) or (R)-camphor-10-sulfonic acid, to obtain one of the diastereomer salts, and if necessary, further subjecting to purification, the resulting diastereomer salt is resolved according to the conventional manner to obtain an optical isomer. Also, when the above reaction is carried out by using the starting compound which has been subjected to optical resolution or separation, a desired optical isomer or geometric isomer can be obtained.

An interesting group of compounds are those compounds of formula (IX) where one or more of the following restrictions apply:
- **Y** is halo, or 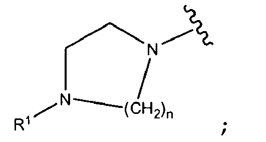
- **R¹** is hydrogen, C₁₋₆alkyl, -C(=O)-R⁷, or a protecting group;
- **n** represents 1 or 2;
- **X** is -SO₂- or -S-;
- **R², R³, R⁴, R⁵, and R⁶** are, each independently, hydrogen, halo, or C₁₋₃alkoxy; or
- **R⁷** is C₁₋₆alkyl or amino.

A further interesting group of compounds are those compounds of formula (IX) where one or more of the following restrictions apply:
- **Y** is fluoro, chloro, or 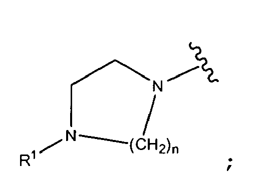
- **R¹** is hydrogen, methyl, ethyl, or a protecting group;
- **n** represents 1 or 2;
- **X** is -SO₂- or -S-; or
- **R², R³, R⁴, R⁵, and R⁶** are, each independently, hydrogen, fluoro, chloro, methoxy, or ethoxy.

A further interesting group of compounds are those compounds of formula (IX) where one or more of the following restrictions apply:
- **Y** is fluoro, chloro, or 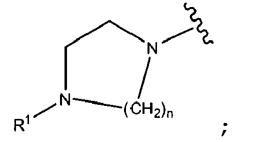
- **R¹** is hydrogen or a protecting group;
- **n** represents 2;
- **X** is -SO₂- or -S-; or
- **R², R³, R⁴, R⁵, and R⁶** are, each independently, hydrogen, chloro, or methoxy.

A further interesting group of compounds are those compounds of formula (IX) where one or more of the following restrictions apply:
- **Y** is fluoro, chloro, or 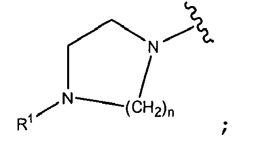
- **R¹** is hydrogen or a protecting group;
- **n** represents 2;
- **X** is -SO₂- or -S-; or
- **R²** is hydrogen;
- **R³** is chloro;
- **R⁴** is hydrogen;
- **R⁵** is methoxy; or
- **R⁶** is methoxy.

A further embodiment of the present invention, relates to a compound of formula (X) *per se* 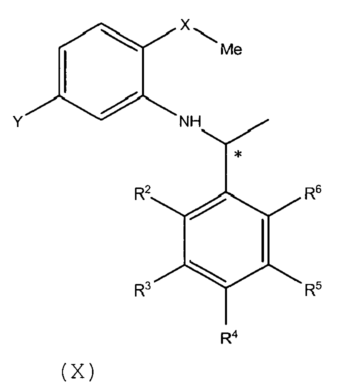 wherein
- **Y**: is halo;
- **X**: is -S-;
- **R², R³, R⁴, R⁵, and R⁶**: are, each independently, hydrogen; halo; C₁₋₃alkoxy; or two adjacent R², R³, R⁴, R⁵, and R⁶ together with the two carbon atoms of the benzene ring to which they are attached form the ring 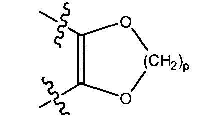 wherein **p** is 2, 3, 4, 5, or 6.

An interesting group of compounds are those compounds of formula (X) where one or more of the following restrictions apply:
- **Y** is halo;
- **X** is -S-;
- **R², R³, R⁴, R⁵, and R⁶** are, each independently, hydrogen, halo, or C₁₋₃alkoxy.

A further interesting group of compounds are those compounds of formula (X) where one or more of the following restrictions apply:
- **Y** is fluoro or chloro;
- **X** is -S-;
- **R², R³, R⁴, R⁵, and R⁶** are, each independently, hydrogen, fluoro, chloro, methoxy or ethoxy.

A further interesting group of compounds are those compounds of formula (X) where one or more of the following restrictions apply:
- **Y** is fluoro;
- **X** is -S-;
- **R², R³, R⁴, R⁵, and R⁶** are, each independently, hydrogen, chloro, or methoxy.

A further interesting group of compounds are those compounds of formula (X) where one or more of the following restrictions apply:
- **Y** is fluoro;
- **X** is -S-;
- **R²** is hydrogen;
- **R³** is chloro;
- **R⁴** is hydrogen;
- **R⁵** is methoxy; or
- **R⁶** is methoxy.

A further embodiment of the present invention, relates to a compound of formula (VI) *per se*, 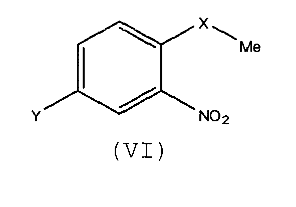 wherein
- **Y**: is 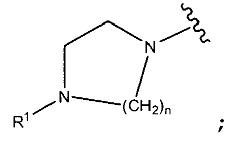
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- n: represents zero, 1, 2, 3, or 4;
- **X**: is -SO₂-; and
- **R⁷**: is C₁₋₆alkyl or amino.

An interesting group of compounds are those compounds of formula (VI) where one or more of the following restrictions apply:
- **Y** is 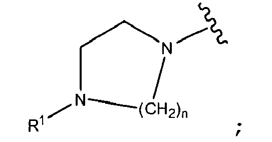
- **R¹** is hydrogen, C₁₋₆alkyl, -C(=O)-R⁷, or a protecting group;
- **n** represents 1 or 2;
- **X** is -SO₂-; or
- **R⁷** is C₁₋₆alkyl or amino.

A further interesting group of compounds are those compounds of formula (VI) where one or more of the following restrictions apply:
- **Y** is 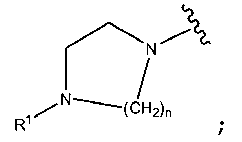
- **R¹** is hydrogen, methyl, ethyl, or a protecting group;
- **n** represents 1 or 2; or
- **X** is -SO₂-.

A further interesting group of compounds are those compounds of formula (VI) where one or more of the following restrictions apply:
- **Y** is 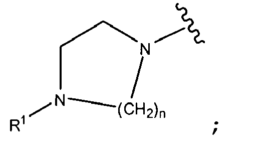
- **R¹** is hydrogen or a protecting group;
- **n** represents 2; or
- **x** is -SO₂-.

The compounds depicted in the present invention, i.e. those compounds encompassed by the formulae (I), (II), (III), (IV), (V), (VI), (VII), (VIII), (IX), (X), (Xa), (Xb), and (Xc), are all useful as intermediates in the preparation of serotonin 5-HT6 antagonists.

As such, a particular embodiment of the present invention relates to the use of compound of formula (IX), as an intermediate in the preparation of a compound of formula (X), (Xa), (Xb), or (Xc), salts, and solvates thereof, 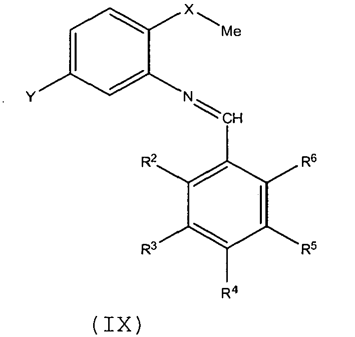 wherein
- **Y**: is halo, or 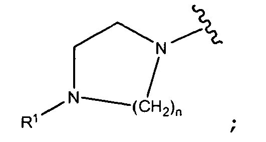
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **x**: is -SO₂- or -S-;
- **R², R³, R⁴, R⁵, and R⁶**: are, each independently, hydrogen; halo; C₁₋₃alkoxy; or two adjacent R², R³, R⁴, R⁵, and R⁶ together with the two carbon atoms of the benzene ring to which they are attached form the ring 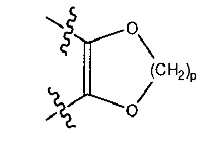 wherein **p** is 2, 3, 4, 5, or 6; and
- **R⁷**: is C₁₋₆alkyl or amino.

A further particular embodiment of the present invention relates to the use of compound of formula (X), as an intermediate in the preparation of a compound of formula (Xa), (Xb, or (Xc), salts, and solvates thereof, 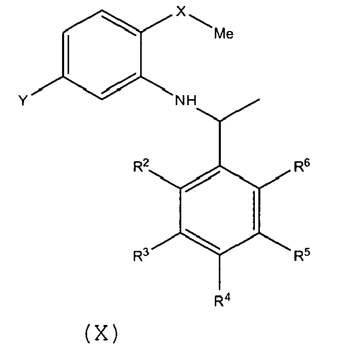 wherein
- **Y**: is halo;
- **x**: is -S-;
- **R²,**: **R³, R⁴, R⁵, and R⁶** are, each independently, hydrogen; halo; C₁₋₃alkoxy; or two adjacent R², R³, R⁴, R⁵, and R⁶ together with the two carbon atoms of the benzene ring to which they are attached form the ring 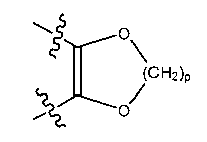 wherein **p** is 2, 3, 4, 5, or 6.

A further particular embodiment of the present invention relates to the use of compound of formula (VI) as an intermediate in the preparation of a compound of formula (X), (Xa), (Xb), or (Xc), salts, and solvates thereof, 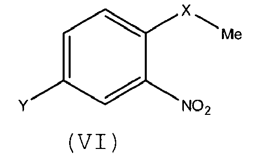 wherein
- **Y**: is 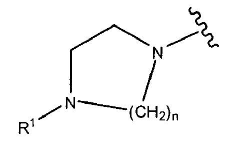
- **R¹**: is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
- **n**: represents zero, 1, 2, 3, or 4;
- **x**: is -SO₂-; and
- **R⁷**: is C₁₋₆alkyl or amino.

The following specific examples are provided to further illustrate the invention and various preferred embodiments.

### Examples

### Example 1

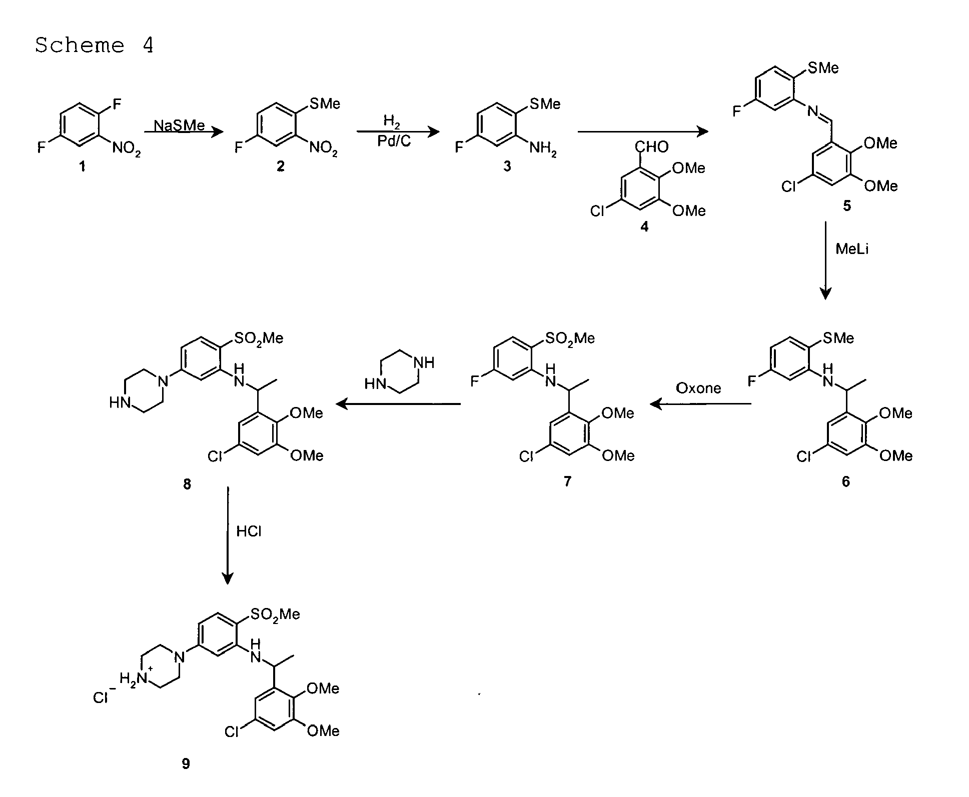

### 4-Fluoro-1-(methylthio)-2-nitrobenzene (2)

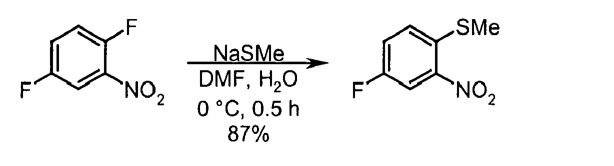

A solution of NaSMe (14.09 g, 180.98 mmol) in H₂O (300 mL) was dropwise added (20 min addition) to a solution of 1,4-difluoro-2-nitrobenzene (28.50 g, 179.19 mmol) in DMF (900 mL) at 0 °C. The mixture was stirred at 0 °C for 0.5 h. The reaction mixture was filtered washing with H₂O (3x150 mL), and the resulting yellow solid was dissolved in CH₂Cl₂ (500 mL). The organic layer was separated, dried over anhydrous Na₂SO₄, filtered and concentrated to give 29.10 g of the title product (R*f*= 0.3 (5% AcOEt/Hexanes), yellow solid, 87% yield).

¹H-NMR (CDCl₃, 300 MHz, δ): 7.99 (ddd, *J* = 8.5, 2.0, 1.2 Hz, 1H, ArH); 7.36 (m, 2H, ArH); 2.51 (s, 3H, CH₃) .

### 5-Fluoro-2-(methylthio)aniline (3)

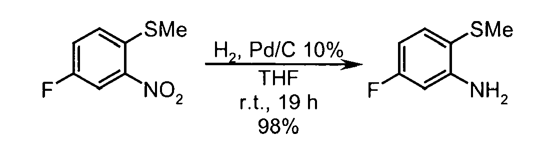

4-Fluoro-1-(methylthio)-2-nitrobenzene (15.00 g, 80.13 mmol) was added to a suspension of Pd/C (7.88 g, 10% weight of Pd on activated carbon, 6.84 mmol) in THF (1.0 L). The reaction mixture was stirred under H₂ (g) atmosphere (balloon) overnight (19 h). It was filtered through Celite® washing with AcOEt (4x500 mL) and the solvent was concentrated off to give 16.70 g of the amine as a brown oil. The crude was purified by flash chromatography on silica gel (25% AcOEt/Hexanes), to yield 12.32 g of the title product (Rf= 0.5 (10% AcOEt/hexanes), orange colored oil, 98% yield).

¹H-NMR (CDCl₃, 250 MHz, δ): 7.34 (m, 1H, ArH); 6.4 (m, 2H, ArH); 4.46 (brs, 2H, NH₂); 2.29 (s, 3H, CH₃).
MS-ES- m/z: 158.3 (M+1).

### N-[1-(5-Chloro-2,3-dimethoxyphenyl)ethyl]-N-[5-fluoro-2-(methylthio)phenyl]amine (6)

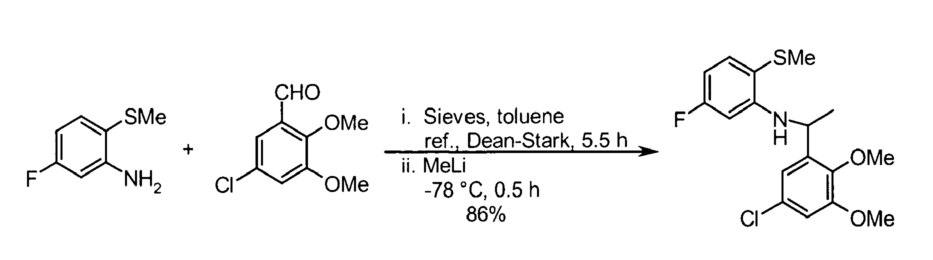

To a solution of the amine (10.0 g, 63.61 mmol) in toluene (140 mL) was added the aldehyde (17.86 g, 89.05 mmol) and 4 Å molecular sieves (32.0 g). After heating at reflux for 5.5 h using a Dean-Stark, the reaction mixture was cooled at -78 °C and MeLi (79.5 mL, 1.6 M solution in Et₂O, 127.22 mmol) was added dropwise (30 min addition). The mixture was stirred at this temperature for 0.5 h, quenched with H₂O (100 mL) and molecular sieves removed by filtration, washing with CH₂Cl₂ (2.0 L). Layers were separated and the aqueous layer was extracted with CH₂Cl₂ (2x75 mL). The combined organic layers were dried over anhydrous Na₂SO₄, filtered and concentrated. The crude residue was flash chromatographed on silica gel (3% AcOEt/Hexanes) to give 19.43 g of the title product (R*f*= 0.7 (10% AcOEt/Hexanes), pale yellow solid, 86% yield).

¹H-NMR (CDCl₃, 250 MHz, δ): 7.36 (dd, *J* = 8.2, 6.6 Hz, 1H, ArH); 6.84 (d, *J* = 2.5 Hz, 1H, ArH); 6.78 (d, *J* = 2.5 Hz, 1H, ArH); 6.28 (td, *J* = 8.5, 2.7 Hz, 1H, ArH); 6.08 (dd, *J* = 11.7, 2.7 Hz, 1H, ArH); 5.52 (d, *J* = 5.2 Hz, 1H, NH); 4.79 (q, *J* = 6.6 Hz, 1H, CH); 3.93 (s, 3H, OCH₃); 3.86 (s, 3H, OCH₃); 2.32 (s, 3H, SCH₃); 1.53 (d, *J* = 6.6 Hz, 3H, CH₃).
MS-ES+ *m*/*z*: 356.2 (M+1).

### N-[1-(5-Chloro-2,3-dimethoxyphenyl)ethyl]-N-[5-fluoro-2-(methylsulfonyl)phenyl]amine (7)

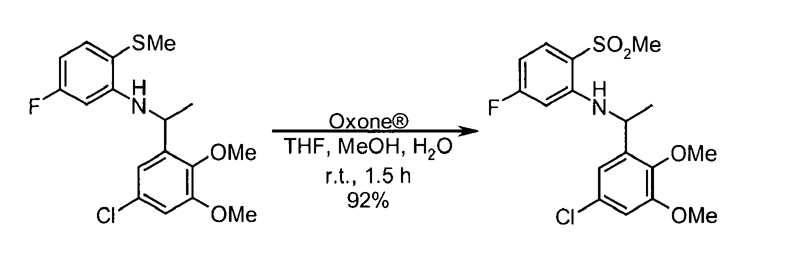

To a suspension of starting material (19.43 g, 54.60 mmol) in THF (100 mL), MeOH (100 mL) and H₂O (100 mL), Oxone® (100.70 g, 163.80 mmol) was added in *ca.* 20 g portions (30 min addition). The mixture was stirred for 1.5 h at room temperature, quenched with H₂O (300 mL) and extracted with AcOEt (3x300 mL). The combined organic layers were dried over anhydrous Na₂SO₄, filtered and concentrated. The crude residue was flash chromatographed on silica gel (20% AcOEt/Hexanes) to give 19.45 g of the title product (R*f*= 0.3 (20% AcOEt/Hexanes), pale yellow solid, 92% yield).

¹H-NMR (CDCl₃, 250 MHz, δ): 7.73 (dd, *J* = 8.8, 6.3 Hz, 1H, ArH); 6.81 (m, 2H, ArH); 6.72 (d, *J* = 4.9 Hz, 1H, NH); 6.42 (td, *J* = 8.5, 2.3 Hz, 1H, ArH); 6.23 (dd, *J* = 11.7, 2.3 Hz, 1H, ArH); 4.82 (q, *J* = 6.5 Hz, 1H, CH); 3.94 (s, 3H, OCH₃); 3.87 (s, 3H, OCH₃); 3.08 (s, 3H, SO₂CH₃); 1.54 (d, 3H, CH₃).
MS-ES+ *m*/*z*: 388.2 (M+1).

### N-(1-(5-Chloro-2,3-dimethoxyphenyl)ethyl)-2-(methylsulfonyl)-5-(piperazin-1-yl)benzenamine (8)

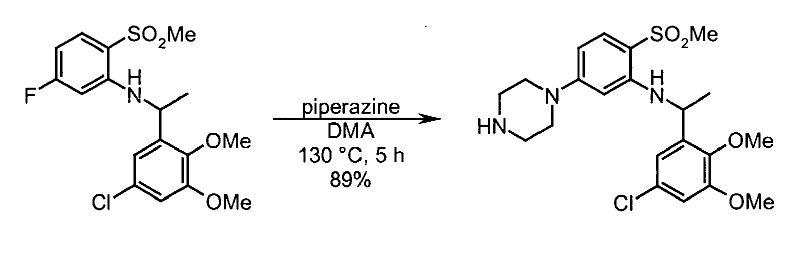

Piperazine (4.51g, 52.36 mmol) was added to a solution of the fluorophenyl derivative (4.04 g, 10.42 mmol) in DMA (40 mL). The reaction mixture was stirred at 130 °C for 5 h, quenched with H₂O (100 mL) and extracted with AcOEt (2x100 mL). The combined organic layers were dried over anhydrous Na₂SO₄ filtered and concentrated. The crude residue was flash chromatographed on silica gel (7% MeOH/CH₂Cl₂) to give 4.20 g of the title product (R*f*= 0.3 (10% MeOH/CH₂Cl₂), white solid, 89% yield).

¹H-NMR (CDCl₃, 250 MHz, δ): 7.48 (d, *J* = 9.1 Hz, 1H, ArH); 6.83 (d, *J* = 2.3 Hz, 1H, ArH); 6.72 (d, *J* = 2.3 Hz, 1H, ArH); 6.46 (d, *J* = 5.5 Hz, 1H, NH); 6.16 (dd, *J* = 9.1, 2.2 Hz, 1H, ArH); 5.87 (d, *J* = 2.2 Hz, 1H, ArH); 4.81 (q, *J* = 6.3 Hz, 1H, CH); 3.84 (s, 3H, OCH₃); 3.79 (s, 3H, OCH₃); 3.66 (m, 4H, CH₂); 3.00 (s, 3H, SO₂CH₃); 2.87 (m, 4H, CH₂); 1.5 (d, *J* = 6.6 Hz, 3H, CH₃) .
MS-ES+ *m*/*z*: 454.3 (M+1).

### [1-(5-Chloro-2,3-dimethoxy-phenyl)-ethyl]-(2-methanesulfonyl-5-piperazin-1-yl-phenyl)-amine hydrochloride [compound 9]

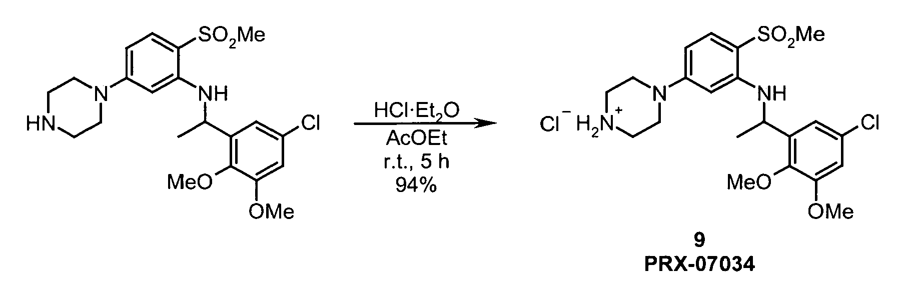

HCl·Et₂O (40.0 mL, 2.0 M solution in Et₂O, 80.00 mmol) was dropwise added to a suspension of starting amine (15.13 g, 33.33 mmol) in AcOEt (340 mL). The reaction mixture was stirred at room temperature for 5 h and the solvent was concentrated off. The resulting solid was suspended in Et₂O (100 mL) and concentrated, to remove excess of HCl. This operation was done for five times, to give 15.32 g of the title product (R*f*= 0.3 (10% MeOH/CH₂Cl₂), white solid, 94% yield).

¹H-NMR (DMSO-d₆, 250 MHz, δ): 9.39 (brs, 2H, NH₂⁺); 7.4 (d, *J* = 8.9 Hz, 1H, ArH); 7.01 (m, 2H, ArH); 6.47 (d, *J* = 6.7 Hz, 1H, NH); 6.36 (dd, *J* = 8.9, 1.6 Hz, 1H, ArH); 5.99 (d, *J* = 1.6 Hz, 1H, ArH); 4.89 (q, *J* = 6.7 Hz, 1H, CH); 3.83 (s, 3H, OCH₃); 3.81 (s, 3H, OCH₃); 3.45 (m, 4H, CH₂); 3.09 (s, 3H, SO₂CH₃); 3.06 (m, 4H, CH₂); 1.5 (d, *J* = 6.7 Hz, 3H, CH₃).
MS-ES+ *m*/*z*: 454.7 (M-HCl).

### Example 2

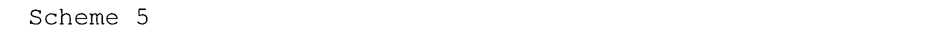

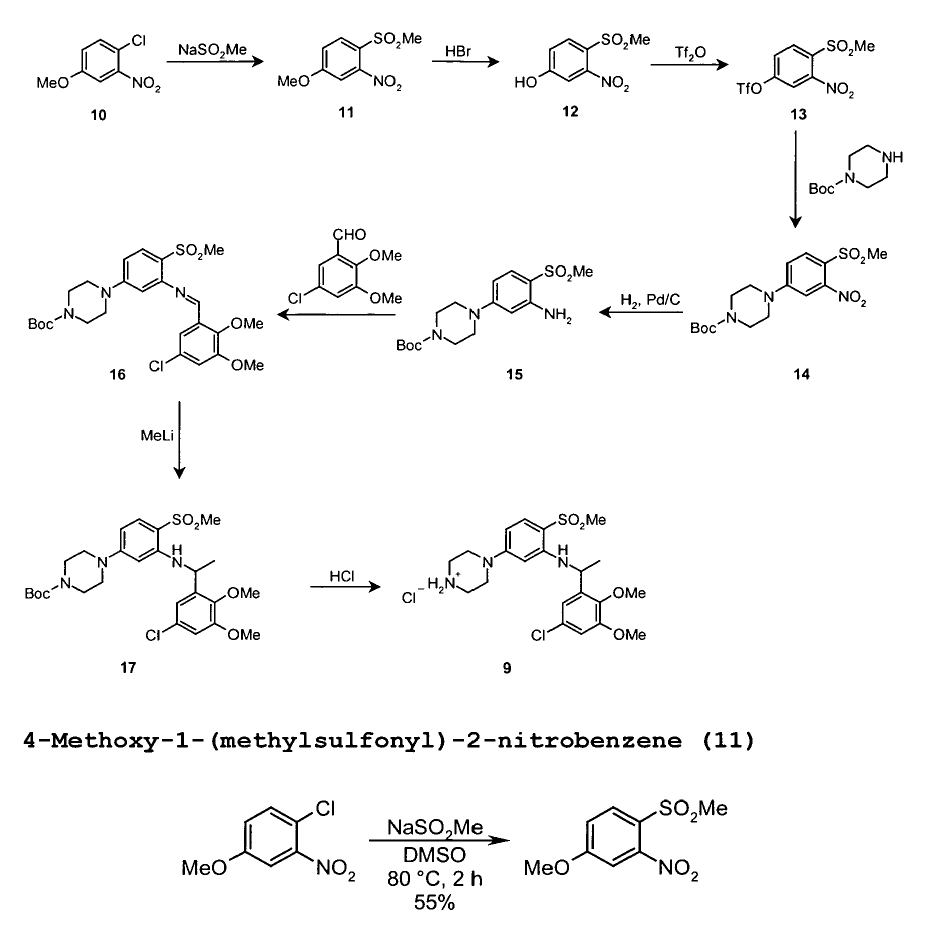

A solution of 1-chloro-4-methoxy-2-nitrobenzene (15.0 g, 79.96 mmol) in DMSO (70 mL) was added dropwise (10 min addition) to a solution of NaSO₂Me (8.57 g, 83.96 mmol) in DMSO (80 mL) at 80 °C, and the mixture was stirred at this temperature for 2 h. The mixture was cooled at room temperature, poured into H₂O (100 mL) and extracted with AcOEt (3x75 mL). The combined organic layers were dried over anhydrous Na₂SO₄, filtered and concentrated. The resulting sulfone was recrystallized from MeOH to give 10.14 g of the title product (R*f*= 0.2 (30% AcOEt/Hexanes), white solid, 55% yield).

¹H-NMR (CDCl₃, 300 MHz, δ): 8.06 (d, *J* = 8.8 Hz, 1H, ArH); 7.28 (d, *J* = 2.3 Hz, 1H, ArH); 7.18 (dd, *J* = 8.8, 2.3 Hz, 1H, ArH); 3.94 (s, 3H, OCH₃) ; 3.36 (s, 3H, SO₂H₃).
MS-EI+ *m*/*z*: 232.1 (M+1).

### 4-(Methylsulfonyl)-3-nitrophenol (12)

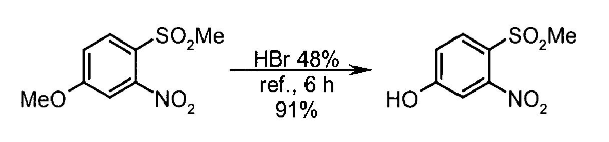

Starting material (9.7 g, 41.95 mmol) was suspended in HBr aq. (48%, 95 mL), and the reaction mixture was refluxed for 6 h. The mixture was cooled to 0 °C, the pH of the solution was adjusted to 13 by adding NaOH aqueous solution (15%) and the aqueous layer was washed with AcOEt (3x60 mL). The pH of the aqueous layer was adjusted to 3 by adding HCl aqueous solution (15%) and it was extracted with AcOEt (3x70 mL). Organic phases were combined, dried over anhydrous Na₂SO₄ and concentrated in vacuo. The residue was washed with Et₂O/MeOH 4:6, affording 8.30 g of the title compound (R*f*= 0.2 (2% MeOH/CH₂Cl₂), white solid, 91% yield).

¹H-NMR (DMSO-d₆, 250 MHz, δ): 7.91 (d, *J* = 8.8 Hz, 1H, ArH); 7.33 (d, *J* = 2.5 Hz, 1H, ArH); 7.21 (dd, *J* = 8.8, 2.5 Hz, 1H, ArH); 3.37 (s, 3H, SO₂CH₃).
MS-EI+ *m*/*z*: 216.3 (M-1).

### 4-(Methylsulfonyl)-3-nitrophenyl trifluoromethanesulfonate (13)

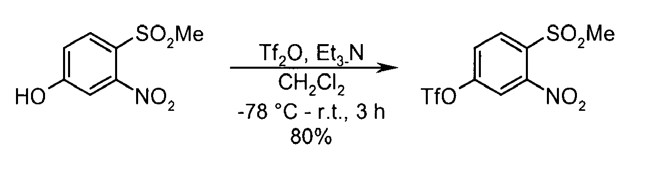

Tf₂O (19.2 mL, 113.56 mmol) was dropwise added to a -78 °C cooled solution of the starting phenol (20.54 g, 94.65 mmol) and Et₃N (39.6 mL, 283.92 mmol) in CH₂Cl₂ (600 mL). The reaction was allowed to warm to r.t. and it was stirred at this temperature for 3 h. The reaction mixture was poured into water (400 mL), layers were separated and aqueous phase extracted with CH₂Cl₂ (2x200 mL). The combined organic layers were dried over anhydrous Na₂SO₄, filtered and concentrated. The crude residue was triturated with 5% MeOH/hexanes (3x100 mL) and 10% MeOH/hexanes (2x100 mL) to furnish 26.30 g of the title product (R*f*= 0.7 (1% MeOH/CH₂Cl₂), white solid, 80% yield).

¹H-NMR (CDCl₃, 250 MHz, δ): 8.35 (d, *J* = 8.7 Hz, 1H, ArH); 7.79 (d, *J* = 2.4 Hz, 1H, ArH); 7.72 (dd, *J* = 8.7, 2.4 Hz, 1H, ArH); 3.47 (s, 3H, SO₂CH₃).
MS-EI- *m*/*z*: 334.4 (M-15), 216.3 (M-Tf).

### tert-butyl 4-[4-(methylsulfonyl)-3-nitrophenyl]piperidine-1-carboxylate (14)

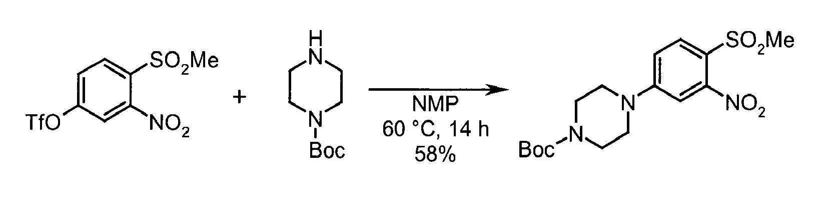

1-Boc-piperazine (19.65 g, 105.50 mmol) was added to a solution of starting triflate (26.30 g, 75.36 mmol) in NMP (240 mL) and the mixture was stirred at 60 °C for 14 h. The reaction mixture was diluted with AcOEt (300 mL) and washed with NH₄Cl saturated aqueous solution (2x250 mL), brine (1x250 mL) and H₂O (1x250 mL). The combined organic layers were dried over anhydrous Na₂SO₄, filtered and concentrated. The aqueous layer was re-extracted with AcOEt (2x200 mL) and the combined organic layers washed with H₂O (1x150 mL) and brine (1x150 mL). The aqueous layers were re-extracted with AcOEt (1x150 mL) and the combined organic phases dried over anhydrous Na₂SO₄, filtered and concentrated. The combined solids were flash chromatographed on silica gel (0.5%→2% MeOH/CH₂Cl₂) to give 16.82 g of the title product (R*f*= 0.3 (40% AcOEt/Hexanes), yellow solid, 58% yield).

¹H-NMR (CDCl₃, 250 MHz, δ): 8.07 (d, *J* = 9.1 Hz, 1H, ArH); 7.26 (d, *J* = 2.7 Hz, 1H, ArH); 7.11 (dd, *J* = 9.1, 2.7 Hz, 1H, ArH); 3.74 (m, 4H, CH₂); 3.56 (m, 4H, CH₂); 3.49 (s, 3H, SO₂CH₃); 1.62 (s, 9H, Boc) .
MS-EI+ *m*/*z*: 403.2 (M+18), 386.1 (M+1), 286.2 (M-Boc).

### tert-Butyl 4-(3-amino-4-(methylsulfonyl)phenyl) piperidine-1-carboxylate (15)

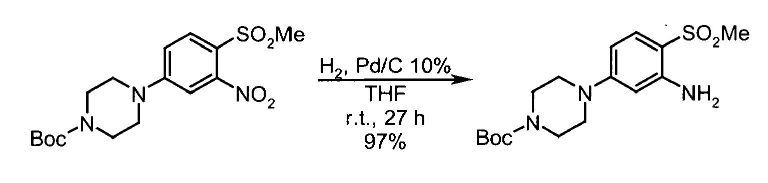

Starting material (11.92 g, 30.96 mmol) was added to a suspension of Pd/C (3.33 g, 10% weight of Pd on activated carbon, 3.10 mmol) in THF (550 mL). The reaction mixture was stirred under H₂ (g) atmosphere (balloon) (ca. 25 h).

Another portion of Pd/C (1.70 g, 10% weight of Pd on activated carbon, 1.55 mmol) suspended in THF (50 mL) was added, and the mixture was stirred under H₂ (g) atmosphere (balloon) for another 2 h. It was filtered through Celite® washing with AcOEt (6x500 mL) and the solvent was concentrated off to yield 10.72 g of the title product (R*f*= 0.3 (5% MeOH/CH₂Cl₂), white solid, 97% yield).

¹H-NMR (CDCl₃, 250 MHz, δ): 7.57 (d, *J* = 9.1 Hz, 1H, ArH); 6.33 (dd, *J* = 9.1, 2.3 Hz, 1H, ArH); 6.07 (d, *J* = 2.3 Hz, 1H, ArH); 4.93 (brs, 2H, NH₂); 3.55 (m, 4H, CH₂); 3.27 (m, 4H, 2XCH₂); 3.02 (s, 3H, SO₂CH₃); 1.48 (s, 9H, Boc) .
MS-EI+ *m*/*z*: 356.3 (M+1), 256.2 (M-Boc).

### tert-Butyl 4-(3-(1-(5-chloro-2,3-dimethoxyphenyl) ethylamino)-4-(methylsulfonyl)phenyl) piperidine-1-carboxylate (17)

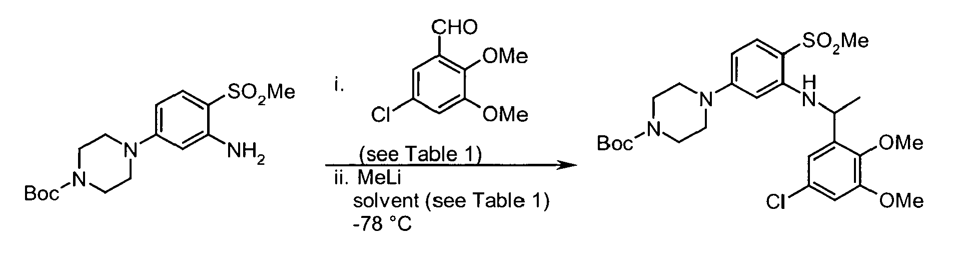

**Table 1**

| **Entry** | **i. conditions** | **ii. solvent** | **Yield (%)** |
|---|---|---|---|
| 1 | MgSO₄, toluene, ref. Dean-Stark, 20 h | THF | 28% |
| 2 | Sieves, toluene, ref. Dean-Stark, 22 h | THF | 35% |
| 3 | Sieves, toluene, ref. Dean-Stark, 20 h | toluene | 32% |
| 5 | Sieves, Et₃N, toluene, ref. Dean-Stark, 19 h | toluene | 29% |

The experimental procedure for Entry 2 is described below:

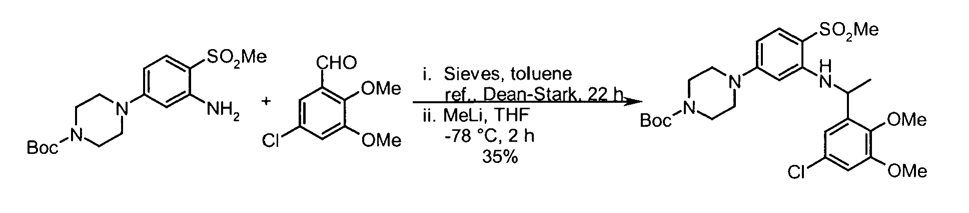

To a solution of starting amine (1.602 g, 4.50 mmol) in toluene (60 mL) was added a solution of aldehyde (0.904 g, 4.50 mmol) in toluene (50 mL) and 4 Å molecular sieves (3.00 g). After heating at reflux for 6 h using a Dean-Stark, another portion of the aldehyde (0.093 g, 0.45 mmol) was added and the mixture stirred at the same temperature for another 16 h. Then, it was quickly concentrated, THF (110 mL) was added and the mixture was cooled at -78 °C. MeLi (8.4 mL, 1.6 M solution in Et₂O, 13.50 mmol) was added dropwise (5 min addition) and the mixture stirred at this temperature for 2 h. The crude mixture was quenched with NH₄Cl saturated aqueous solution (50 mL). Molecular sieves were filtered, mother liquors were extracted with AcOEt (4x100 mL) and the combined organic layers dried over anhydrous Na₂SO₄, filtered and concentrated. The crude was purified by medium flash chromatographed (Biotage system SP1, 15%→50% AcOEt/Hexanes) to give 0.863 g of the title product (R*f*= 0.3 (2% MeOH/ CH₂Cl₂), white solid, 35% yield).

¹H-NMR (CDCl₃, 250 MHz, δ): 7.56 (s, 1H, ArH); 6.86 (d, *J* = 2.3 Hz, 1H, ArH); 6.76 (d, *J* = 2.3 Hz, 1H, ArH); 6.54 (d, *J* = 6.0 Hz, 1H, NH); 6.24 (dd, *J* = 8.9, 1.9 Hz, 1H, ArH); 5.97 (d, *J* = 1.9 Hz, 1H, ArH); 4.85 (q, *J* = 6.0 Hz, 1H, CH); 3.89 (s, 3H, OCH₃); 3.85 (s, 3H, OCH₃); 3.47 (m, 4H, CH₂); 3.18 (m, 4H, CH₂); 3.04 (s, 3H, SO₂CH₃); 1.54 (d, *J* = 6.0 Hz, 3H, CH₃); 1.47 (s, 9H, Boc).
MS-ES+ *m*/*z*: 554.8 (M+1).

### [1-(5-Chloro-2,3-dimethoxy-phenyl)-ethyl]-(2-methanesulfonyl-5-piperazin-1-yl-phenyl)-amine hydrochloride [compound (9)]

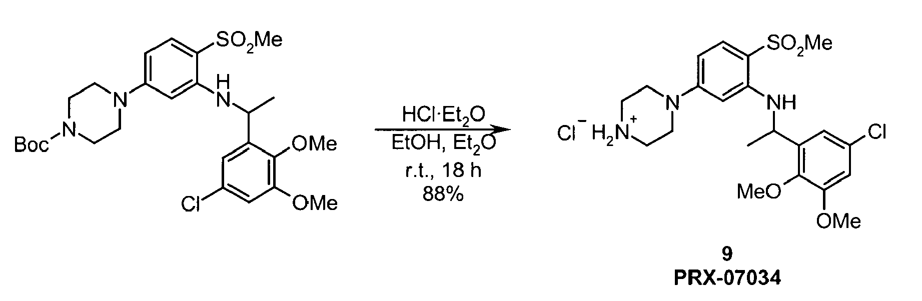

HCl·Et₂O (19.6 mL, 2.0 M solution in Et₂O, 3.89 mmol) was dropwise added to a suspension of starting amine (2.15 g, 3.89 mmol) in EtOH (20 mL) and Et₂O (20 mL). The reaction mixture was stirred at room temperature for 16 h. HCl·Et₂O (19.6 mL, 2 M solution in Et₂O, 3.89 mmol) was added and the mixture stirred for another 2 h till starting material disappeared. The solvent was concentrated off and the resulting solid was suspended in Et₂O (15 mL) and concentrated, in order to remove excess of HCl. This operation was done for three times, to give 1.68 g of the title product (R*f*= 0.3 (10% MeOH/CH₂Cl₂), white solid, 88% yield).

¹H-NMR (DMSO-d₆, 250 MHz, δ): 9.39 (brs, 2H, NH₂⁺); 7.4 (d, *J* = 8.9 Hz, 1H, ArH); 7.01 (m, 2H, ArH); 6.47 (d, *J* = 6.7 Hz, 1H, NH); 6.36 (dd, *J* = 8.9, 1.6 Hz, 1H, ArH); 5.99 (d, *J* = 1.6 Hz, 1H, ArH); 4.89 (q, *J* = 6.7 Hz, 1H, CH); 3.83 (s, 3H, OCH₃) ; 3.81 (s, 3H, OCH₃); 3.45 (m, 4H, CH₂); 3.09 (s, 3H, SO₂CH₃); 3.06 (m, 4H, CH₂); 1.5 (d, *J* = 6.7 Hz, 3H, CH₃).
MS-ES+ *m*/*z*: 454.7 (M-HCl).

### Example 3

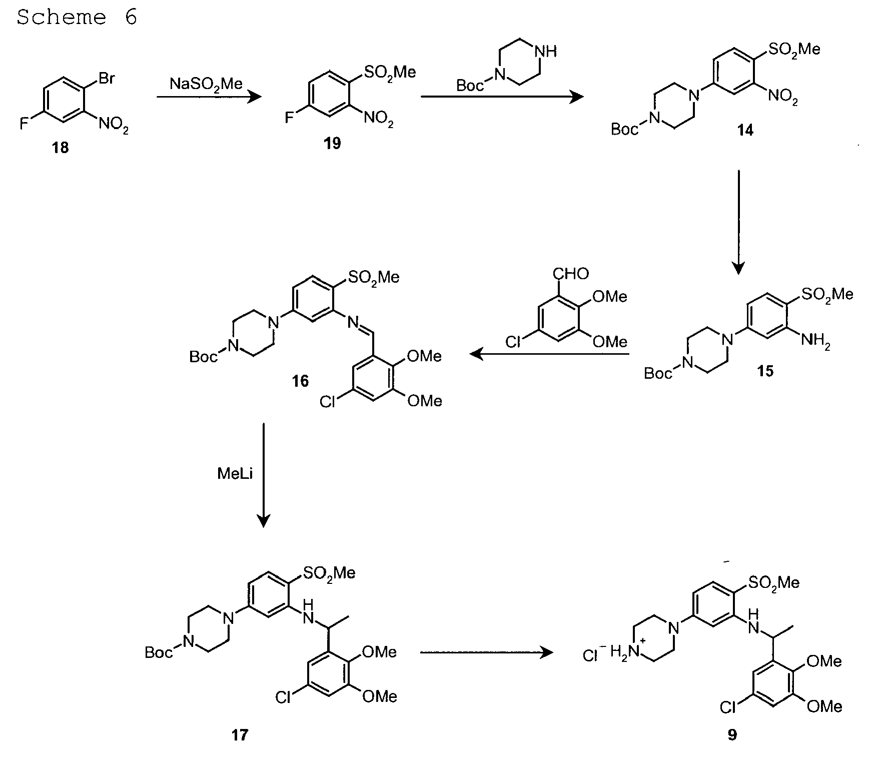

### 4-Fluoro-1-(methylsulfonyl)-2-nitrobenzene (19)

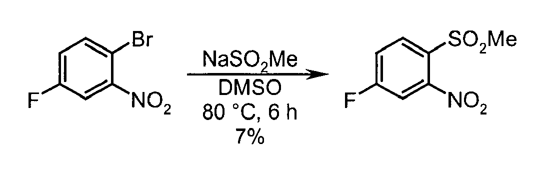

A solution of 1-bromo-4-fluoro-2-nitrobenzene (3.0 g, 13.64 mmol) in DMSO (5 mL) was dropwise added (10 min addition) to a solution of NaSO₂Me (1.96 g, 16.32 mmol) in DMSO (5 mL) at 80 °C and the mixture was stirred at this temperature for 6 h. The reaction mixture was quenched with H₂O (35 mL) and the slurry heated at 80 °C for 0.5 h. The solid was filtered, washing with H₂O (3x10 mL) to give 1.66 g of the title product as a yellow solid. Mother liquors were extracted with AcOEt (3x20 mL) and the combined organic layers washed with brine (3x15 mL). The organic layer was dried over anhydrous Na₂SO₄, filtered and concentrated to give 0.79 g of the title product. MeOH (20 mL) was added to the combined solids and the mixture filtered. The filtrated were concentrated and the crude mixture was flash chromatographed on silica gel (CH₂Cl₂) to give 0.22 g of the title product (R*f*= 1.0 (CH₂Cl₂), yellow solid, 7% yield).

¹H-NMR (CDCl₃, 250 MHz, δ): 8.24 (dd, *J* = 8.8, 5.2 Hz, 1H, ArH); 7.58 (dd, *J* = 7.2, 2.4 Hz, 1H, ArH); 7.48 (ddd, *J* = 8.8, 7.2, 2.4 Hz, 1H, ArH); 3.43 (s, 3H, SO₂CH₃).

### tert-Butyl 4-[4-(methylsulfonyl)-3-nitrophenyl]piperidine-1-carboxylate (14)

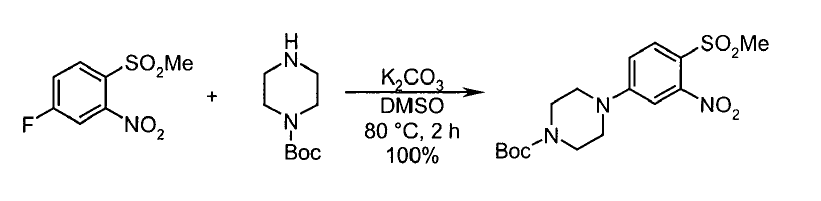

K₂CO₃ (0.095 g, 0.68 mmol) and 1-Boc-piperazine (0.170 g, 0.91 mmol) was added to a solution of starting fluoride (0.100 g, 0.46 mmol) in DMSO (2.0 mL) and the mixture was stirred at 80 °C for 2 h. It was allowed to reach room temperature, diluted with AcOEt (15 mL) and washed with H₂O (2x20 mL) and brine (2x20 mL). The organic layer was dried over anhydrous Na₂SO₄, filtered and concentrated to yield 0.176 g of the title product (R*f*= 0.3 (1% MeOH/CH₂Cl₂), yellow solid, 100% yield).

¹H-NMR (CDCl₃, 250 MHz, δ): 8.07 (d, *J* = 9.1 Hz, 1H, ArH); 7.26 (d, *J* = 2.7 Hz, 1H, ArH); 7.11 (dd, *J* = 9.1, 2.7 Hz, 1H, ArH); 3.74 (m, 4H, CH₂); 3.56 (m, 4H, CH₂); 3.49 (s, 3H, SO₂CH₃); 1.62 (s, 9H, Boc).
MS-EI+ m/z: 403.2 (M+18), 386.1 (M+1), 286.2 (M-Boc).

### tert-Butyl 4-(3-(1-(5-chloro-2,3-dimethoxyphenyl)ethylamino)-4-(methylsulfonyl)phenyl)piperidine-1-carboxylate (17)

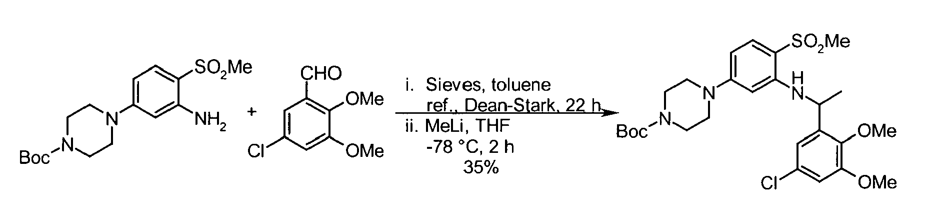

To a solution of starting amine (1.602 g, 4.50 mmol) in toluene (60 mL) was added a solution of aldehyde (0.904 g, 4.50 mmol) in toluene (50 mL) and 4 Å molecular sieves (3.00 g). After heating at reflux for 6 h using a Dean-Stark, another portion of the aldehyde (0.093 g, 0.45 mmol) was added and the mixture stirred at the same temperature for another 16 h. Then, it was quickly concentrated, THF (110 mL) was added and the mixture was cooled at -78 °C. MeLi (8.4 mL, 1.6 M solution in Et₂O, 13.50 mmol) was added dropwise (5 min addition) and the mixture stirred at this temperature for 2 h. The crude mixture was quenched with NH₄Cl saturated aqueous solution (50 mL). Molecular sieves were filtered, mother liquors were extracted with AcOEt (4x100 mL) and the combined organic layers dried over anhydrous Na₂SO₄, filtered and concentrated. The crude was purified by medium flash chromatographed (Biotage system →50% SP1, 15% AcOEt/Hexanes) to give 0.863 g of the title product (R*f*= 0.3 (2% MeOH/ CH₂Cl₂), white solid, 35% yield) .
¹H-NMR (CDCl₃, 250 MHz, δ): 7.56 (s, 1H, ArH); 6.86 (d, *J* = 2.3 Hz, 1H, ArH); 6.76 (d, *J* = 2.3 Hz, 1H, ArH); 6.54 (d, *J* = 6.0 Hz, 1H, NH); 6.24 (dd, *J* = 8.9, 1.9 Hz, 1H, ArH); 5.97 (d, *J* = 1.9 Hz, 1H, ArH); 4.85 (q, *J* = 6.0 Hz, 1H, CH); 3.89 (s, 3H, OCH₃); 3.85 (s, 3H, OCH₃); 3.47 (m, 4H, CH₂); 3.18 (m, 4H, CH₂); 3.04 (s, 3H, SO₂CH₃); 1.54 (d, *J* = 6.0 Hz, 3H, CH₃); 1.47 (s, 9H, Boc).
MS-ES+ *m*/*z*: 554.8 (M+1).

### [1-(5-Chloro-2,3-dimethoxy-phenyl)-ethyl]-(2-methanesulfonyl-5-piperazin-1-yl-phenyl)-amine hydrochloride [compound (9)]

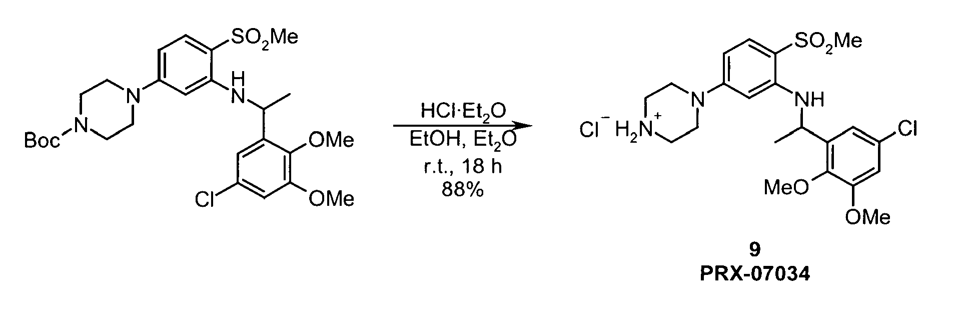

HCl·Et₂O (19.6 mL, 2.0 M solution in Et₂O, 3.89 mmol) was dropwise added to a suspension of starting amine (2.15 g, 3.89 mmol) in EtOH (20 mL) and Et₂O (20 mL). The reaction mixture was stirred at room temperature for 16 h. HCl·Et₂O (19.6 mL, 2 M solution in Et₂O, 3.89 mmol) was added and the mixture stirred for another 2 h till starting material disappeared. The solvent was concentrated off and the resulting solid was suspended in Et₂O (15 mL) and concentrated, in order to remove excess of HCl. This operation was done for three times, to give 1.68 g of the title product (R*f*= 0.3 (10% MeOH/CH₂Cl₂), white solid, 88% yield).

¹H-NMR (DMSO-d₆, 250 MHz, δ): 9.39 (brs, 2H, NH₂⁺); 7.4 (d, *J* = 8. 9 Hz, 1H, ArH); 7.1 (m, 2H, ArH); 6.47 (d, *J* = 6. 7 Hz, 1H, NH); 6.36 (dd, *J* = 8.9, 1.6 Hz, 1H, ArH); 5.99 (d, *J* = 1.6 Hz, 1H, ArH); 4.89 (q, *J* = 6.7 Hz, 1H, CH); 3.83 (s, 3H, OCH₃); 3.81 (s, 3H, OCH₃); 3.45 (m, 4H, CH₂); 3.09 (s, 3H, SO₂CH₃); 3.06 (m, 4H, CH₂); 1.5 (d, *J* = 6.7 Hz, 3H, CH₃).
MS-ES+ *m*/*z*: 454.7 (M-HCl).

### Example 4

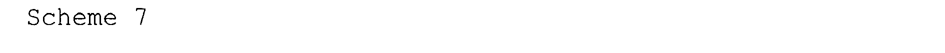

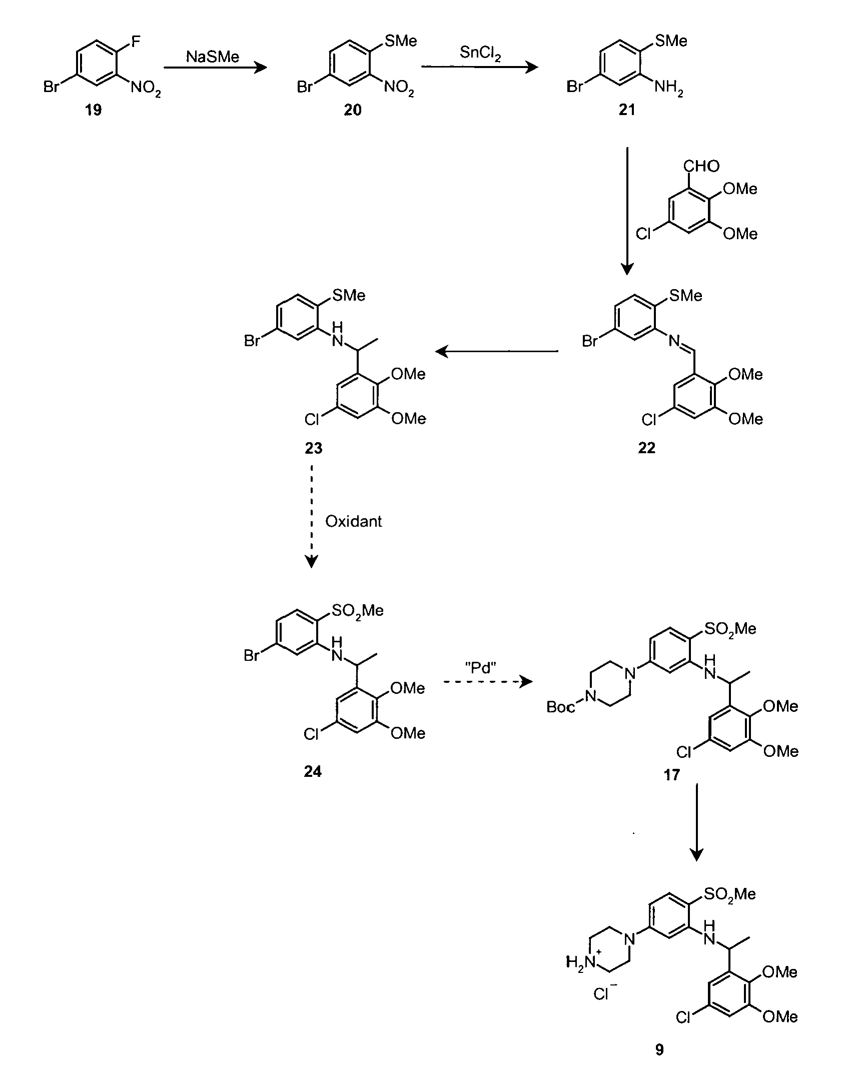

### 4-Bromo-1-(methylthio)-2-nitrobenzene (20)

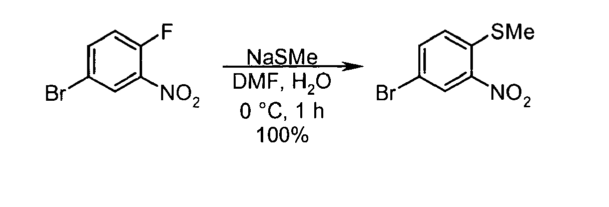

A solution of NaSMe (0.81 g, 10.37 mmol) in H₂O (17 mL) was added dropwise (10 min addition) to a solution of 4-bromo-1-fluoro-2-nitrobenzene (2.14 g, 9.43 mmol) in DMF (50 mL) at 0 °C and the mixture was stirred at this temperature for 1 h. The reaction mixture was filtered, washing with H₂O (3x75 mL) to give 2.05 g of the title product as a yellow solid. Brine (100 mL) was added to mother liquors and these were extracted with AcOEt (2x150 mL). The combined organic layers were dried over anhydrous Na₂SO₄, filtered and concentrated to give 0.293 g of the title product. The combination of both solids gave 2.34 g of the product (R*f*= 0.3 (10% AcOEt/hexanes), yellow solid, 100% yield).

¹H-NMR (CDCl₃, 250 MHz, δ): 8.43 (d, *J* = 2.2 Hz, 1H, ArH); 7.72 (dd, *J* = 8.8, 2.2 Hz, 1H, ArH); 7.29 (s, 1H, ArH); 2.53 (s, 3H, SCH₃).

### 5-Bromo-2-(methylthio)aniline (21)

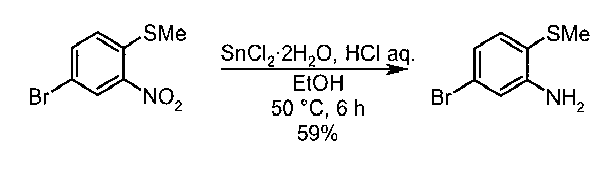

A solution of SnCl₂·2H₂O (1.62 g, 7.06 mmol) in HCl aq. 37% (2.9 mL) was added to a suspension of 4-bromo-1-(methylthio)-2-nitrobenzene (0.50 g, 2.02 mmol) in EtOH (11.0 mL). The reaction mixture was stirred at 50 °C for 6 h. Then, it was poured into NaOH aqueous solution (36%) and the mixture was taken to pH= 10 and extracted with AcOEt (60 mL). The organic layer was washed with H₂O (2x60 mL), dried over anhydrous Na₂SO₄, filtered and concentrated. The crude residue was flash chromatographed on silica gel (5% AcOEt/hexanes) to give 0.26 g of the title product (R*f*= 0.5 (10% AcOEt/ hexanes), yellow solid, 59% yield).

¹H-NMR (CDCl₃, 250 MHz, δ): 7.19 (d, *J* = 8.0 Hz, 1H, ArH); 6.86 (d, *J* = 2.1 Hz, 1H, ArH); 6.81 (dd, *J* = 8.0, 2.1 Hz, 1H, ArH); 4.34 (brs, 2H, NH₂); 2.32 (s, 3H, SCH₃).

### N-[5-Bromo-2-(methylthio)phenyl]-N-[1-(5-chloro-2,3-dimethoxyphenyl)ethyl]amine (22)

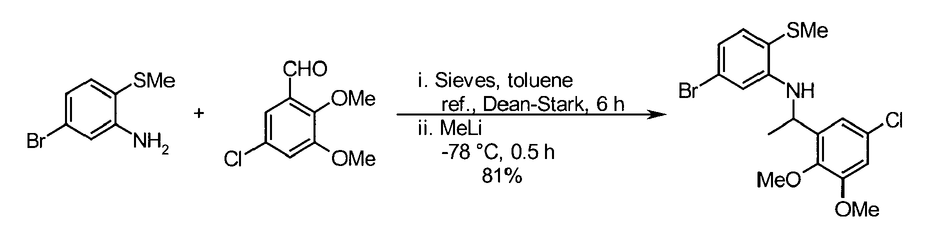

To a solution of starting amine (0.213 g, 0.98 mmol) in toluene (5.0 mL) was added aldehyde (0.255 g, 1.27 mmol) and 4 Å molecular sieves (2.00 g). After heating at reflux for 6 h using a Dean-Stark, the reaction mixture was cooled at -78 °C and MeLi (1.2 mL, 1.6 M solution in Et₂O, 1.92 mmol) added dropwise. The mixture was stirred at this temperature for 0.5 h, quenched with H₂O (10 mL) and molecular sieves were removed by filtration. The mother liquors were extracted with AcOEt (3x10 mL). The combined organic layers were dried over anhydrous Na₂SO₄, filtered and concentrated. The crude residue was flash chromatographed on silica gel (4% AcOEt/Hexanes) to give 0.330 g of the title product (R*f*= 0.7 (10% AcOEt/Hexanes), pale yellow solid, 81% yield).

¹P-NMR (CDCl₃, 250 MHz, δ): 7.22 (d, *J* = 8.0 Hz, 1H, ArH); 6.84 (d, *J* = 2.2 Hz, 1H, ArH); 6.78 (d, *J* = 2.2 Hz, 1H, ArH); 6.72 (dd, *J* = 8.0, 2.0 Hz, 1H, ArH); 6.52 (d, *J* = 2.0 Hz, 1H, ArH); 5.35 (d, *J* = 6.5 Hz, 1H, NH); 4.82 (q, *J* = 6.5 Hz, 1H, CH); 3.95 (s, 3H, OCH₃); 3.87 (s, 3H, OCH₃); 2.34 (s, 3H, SCH₃); 1.51 (d, *J* = 6.5 Hz, 3H, CH₃).

### Example 5

The synthesis of ketone **3** was carried out by reducting the commercially available aldehyde **1** to the corresponding alcohol **2** following the procedure disclosed in WO06/081332A1, followed by the oxidation to ketone **3** with MnO₂ with a good yield (Scheme 8).

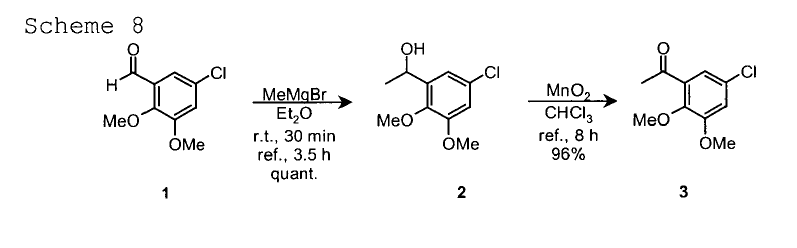

The commercially available aldehyde 1 and ketone 3 was submitted to a reductive amination as shown in Scheme 9 and Table 2. As it can be observed from entries 1 and 2, ketone 3 did not lead to the desired product under any of the tested conditions, recovering the two starting compounds; however, the aldehyde 1 reacted with good yield, at scale of 10 g of 43, to give compound 44 (see entry 3).

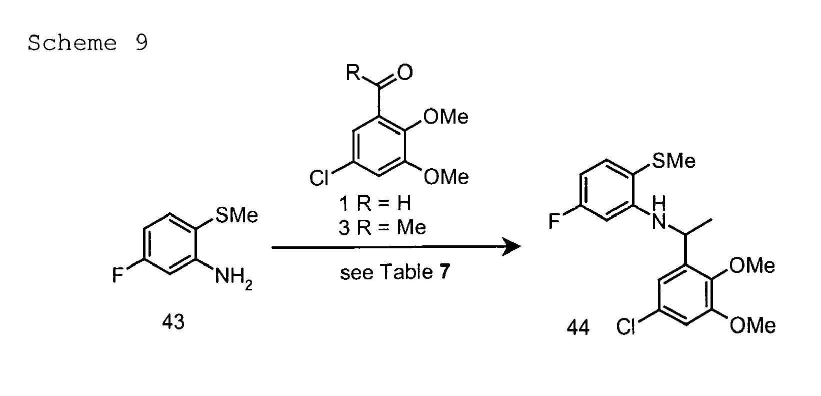

**Table 2**

| Entry | R | Conditions | Yield (%) |
|---|---|---|---|
| 1 | Me | 1) AcOH, CH₂Cl₂, r.t., 7 h 2) NaB(OAc)₃H, r.t., 14 h | - |
| 2 | Me | 1) Sieves, toluene, ref., 3h 2) H₂, Pd/C, THF, r.t., 14 h | - |
| 3 | H | 1) Sieves, toluene, ref., Dean-Stark, 5.5 h 2) MeLi, - 78 °C, 0.5 h | 86% |

### Example 6

The synthesis of the intermediate amine **43** was successfully carried out in two steps, as shown in Scheme 10 starting with the commercially available difluoro derivative **41.**

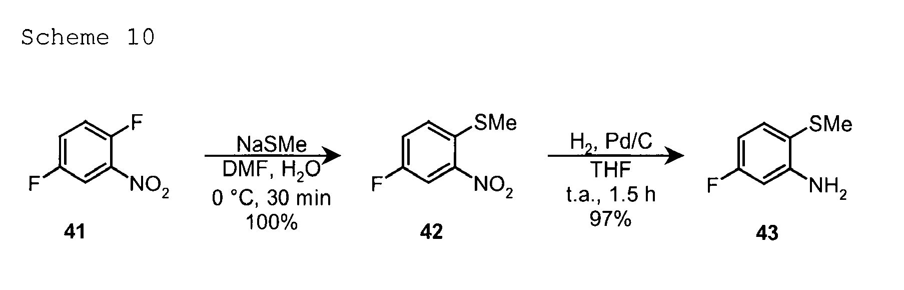

### Example 7

The oxidation of thioether to sulphone was carried out with a good yield using both *m*-CPBA in CHCl₃ at r.t. (83%) and Oxone® in THF/MeOH/H₂O at r.t. (83%) or H₂O₂ in AcOH at 100 °C (77%), at scale of 100 mg. It was scaled with 1.0 g of thioether **44** using Oxone®, since those conditions turned out to be those with a better yield and more comfortable for the production and purification, thereby obtaining a yield of the sulphone **45** of 86% (Scheme 11).

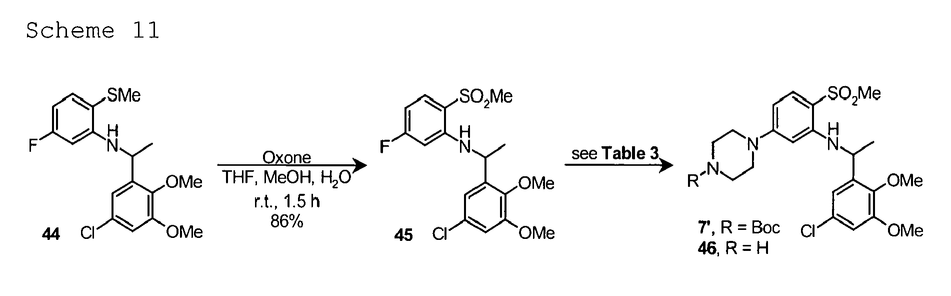

### Example 8 Synthesis of 5-bromo-2-(methylthio)aniline (compound 52)

An alternative proposal involves obtaining the intermediate **52,** the synthesis of which is carried out in two steps as shown in scheme 12.

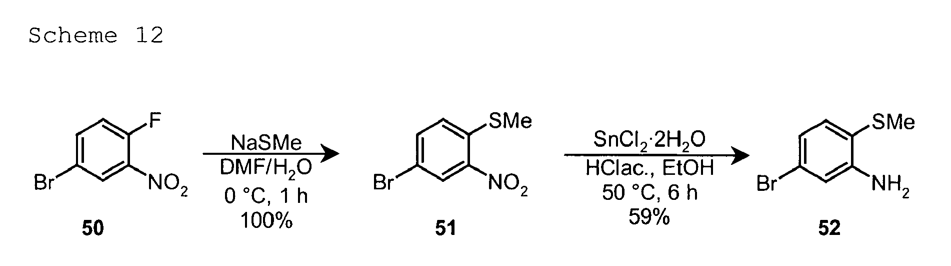

The intermediate **52** was successfully obtained.
The following compounds **53 and 54** are obtained through a Buchwald coupling reaction with piperazine (Scheme 13).

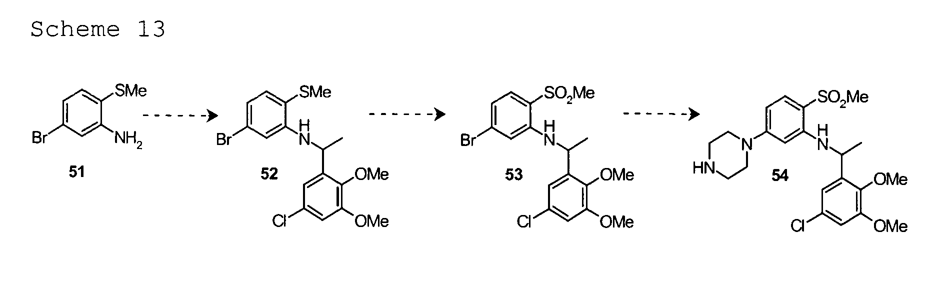

## Claims

1. A process for the preparation of a compound of formula (X), and pharmaceutically acceptable salts and solvates thereof, 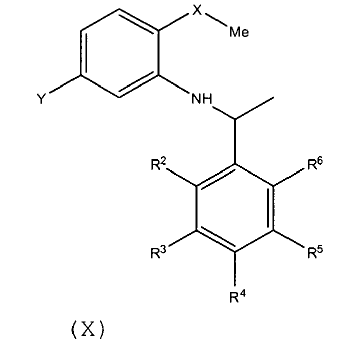 said process comprising the reaction of a compound of formula (IX) with a methyl nucleophile in an organic solvent at a temperature between -100 and 50°C; 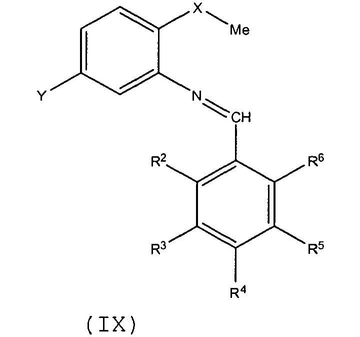 wherein in each of the compounds of formula (IX) and (X), independently,
**Y** is halo or 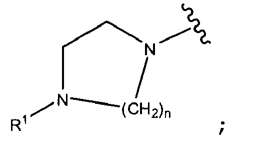
**R¹** is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
**n** represents zero, 1, 2, 3, or 4;
**X** is -SO₂- or -S-;
**R², R³, R⁴, R⁵, and R⁶** are, each independently, hydrogen; halo; C₁₋₃alkoxy; or two adjacent substituents of the group of R², R³, R⁴, R⁵, and R⁶, together with the two carbon atoms of the benzene ring to which they are attached to, form the ring 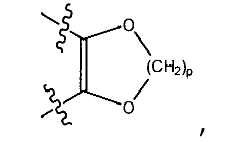 wherein **p** is 2, 3, 4, 5, or 6; and
**R⁷** is C₁₋₆alkyl or amino.

2. A process for the preparation of a compound of formula (IX) as defined in claim 1, said process comprising the reaction of a compound of formula (VII) with a compound of formula (VIII) under dehydrating conditions, optionally using a drying agent, and optionally in the presence of a catalyst, 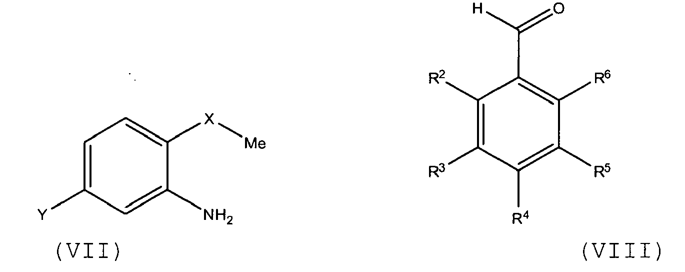 wherein
**Y, X, R², R³, R⁴, R⁵, and R⁶** are as defined in claim 1.

3. The process according to claim 1, wherein compound of formula (IX) is obtained by reacting a compound of formula (VII) with a compound of formula (VIII) under dehydrating conditions, optionally using a drying agent, and optionally in the presence of a catalyst; 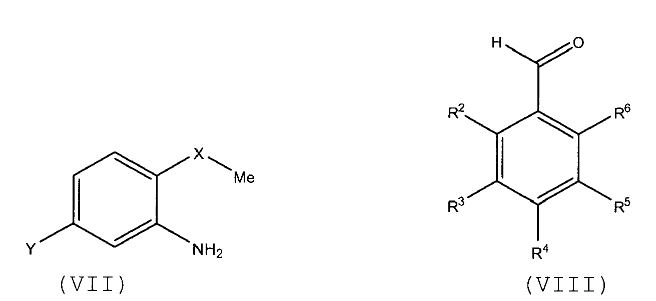 wherein
**Y, X, R², R³, R⁴, R⁵, and R⁶** are as defined in claim 1.

4. A process for the preparation of a compound of formula (X) as defined in claim 1, said process comprising the reaction of a compound of formula (VII) with a compound of formula (VIII) under dehydrating conditions, optionally using a drying agent, and optionally in the presence of a catalyst, 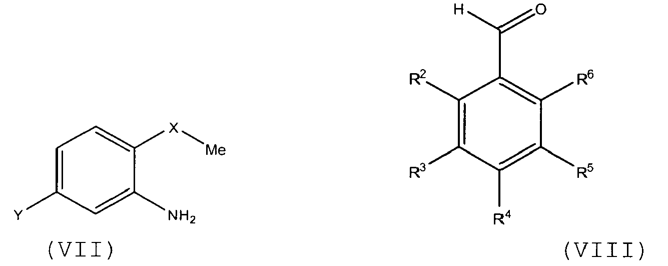 thereby obtaining compound of formula (IX) 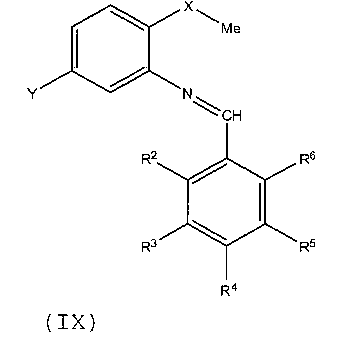 followed by the addition to the reaction mixture of a methyl nucleophile in an organic solvent at a temperature between -100 and 50°C; wherein
**Y, X, R², R³, R⁴, R⁵, and R⁶** are as defined in claim 1;

5. The process according to any one of claims 2-4, wherein compound of formula (VII) is obtained by treating a compound of formula (VI) with a reducing agent in an organic solvent at a temperature between -20 and 80° C, 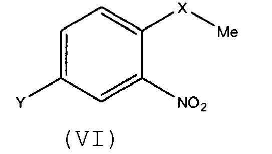 wherein
**Y and X** are as defined in claim 1.

6. The process according to claim 1, wherein when X is -S- in compound of formula (X), this compound is further reacted with an oxidant in a solvent selected from water, one or more organic solvents, and mixtures thereof, thereby obtaining compound of formula (Xa), and pharmaceutically acceptable salts and solvates thereof, 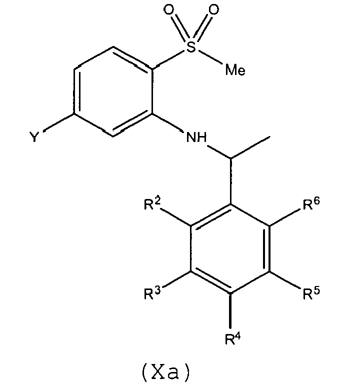 wherein
**Y, R², R³, R⁴, R⁵, and R⁶** are as defined in claim 1.

7. The process according to any one of claims 1 or 6, wherein when Y is halo in compound of formula (X) or (Xa), this compound is further reacted with compound of formula (IV) in an organic solvent, optionally in the presence of a base, at a temperature between 20 and 150 °C, 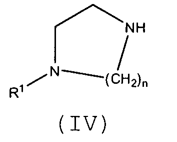 wherein
**R¹ and n** are as defined in claim 1;
thereby obtaining compound of formula (Xb) or (Xc), respectively, and pharmaceutically acceptable salts and solvates thereof, 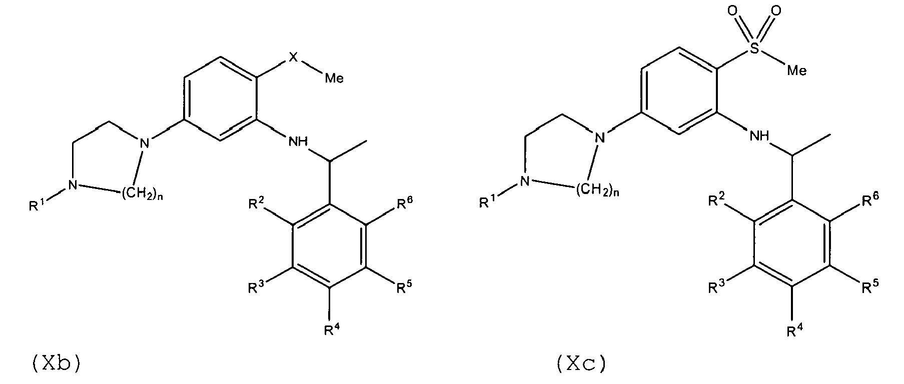 wherein
**X, R¹, n, R², R³, R⁴, R⁵, and R⁶** are, each independently, as defined in claim 1.

8. The process according to claim 7, wherein when X is -S- in compound of formula (Xb), this compound is further reacted with an oxidant in a solvent selected from water, one or more organic solvents, and mixtures thereof, thereby obtaining compound of formula (Xc), and pharmaceutically acceptable salts and solvates thereof, 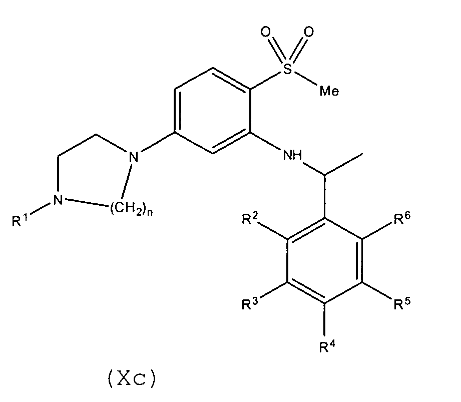 wherein
**R¹, n, R², R³, R⁴, R⁵, and R⁶** are, each independently, as defined in claim 1.

9. The process according to any one of claims 1, 4, 6, 7, or 8, wherein compound of formula (X), (Xa), (Xb), or (Xc) is further reacted with an acid in an organic solvent, thereby obtaining a salt of compound of formula (X), (Xa), (Xb), or (Xc), respectively.

10. The process according to claim 5, wherein when Y is 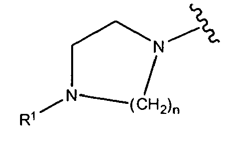 and X is -SO₂- in compound of formula (VI),
said compound is obtained by reacting a compound of formula (III) with compound of formula (IV) as defined in claim 7, in an organic solvent, optionally in the presence of a base, at a temperature between 20 and 150 °C; 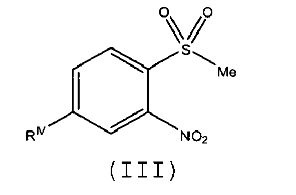 wherein
**R^{IV}** is halo, or a sulfonate selected from nonaflate, tresylate, nosylate, mesylate, tosylate, brosylate, and triflate.

11. The process according to claim 10, wherein when R^{IV} is a sulfonate in compound of formula (III), said compound is obtained by deprotecting compound of formula (II) and reacting the resultant hydroxyl group with a sulfonylating agent, in an organic solvent, in the presence of a base, 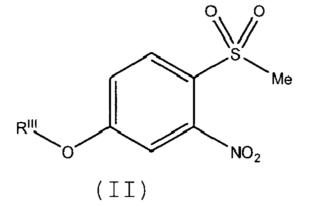 wherein
**R^{III}** is C₁₋₆alkyl, benzyl, silyl, p-methoxybenzyl, or - C(=O)-R^{v}; and
**R^{v}** is hydrogen, C₁₋₆alkyl, phenyl, o-tolyl, *m*-tolyl, *p-*tolyl, or *p*-nitrophenyl.

12. The process according to claim 10 or 11, wherein when R^{IV} is halo in compound of formula (III), each of compounds of formula (III) and (II) is obtained by reacting a compound of formula (I) with a sulphur nucleophile in an organic solvent, at a temperature between 50 and 150°C 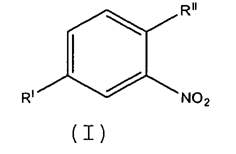 wherein
**R^{I}** is halo or -OR^{III};
**R^{II}** is halo;
**R^{III}** is C₁₋₆alkyl, benzyl, silyl, p-methoxybenzyl, or - C(=O)-R^{v}; and
**R^{V}** is hydrogen, C₁₋₆alkyl, phenyl, *o*-tolyl, *m*-tolyl, *p-*tolyl, or p-nitrophenyl.

13. The process according to claim 5, wherein where X is -S- and Y is halo in compound of formula (VI), said compound is obtained by reacting compound of formula (V) with a methyl-bearing sulphur nuclophile, in a mixture of an organic solvent and water, at a temperature between -40 and 150 °C, 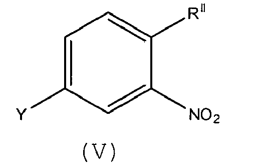 wherein
**Y** is halo; and
**R^{II}** is halo.

14. A compound of formula (IX) 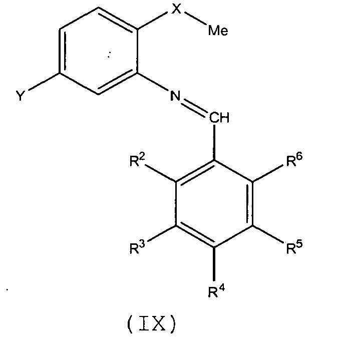 wherein
**Y** is halo, or 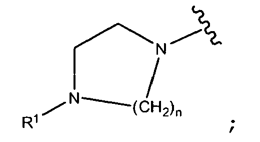
**R¹** is hydrogen, C₁₋₆alkyl optionally substituted with C₃₋₇ cycloalkyl, phenyl optionally substituted with C₁₋₆alkyl or C₁₋₆alkoxy, -C(=O)-R⁷, or a protecting group;
**n** represents zero, 1, 2, 3, or 4;
**X** is -SO₂- or -S-;
**R², R³, R⁴, R⁵, and R⁶** are, each independently, hydrogen; halo; C₁₋₃alkoxy; or two adjacent R², R³, R⁴, R⁵, and R⁶ together with the two carbon atoms of the benzene ring to which they are attached form the ring 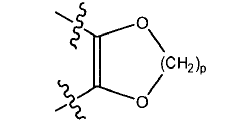 wherein **p** is 2, 3, 4, 5, or 6; and
**R⁷** is C₁₋₆alkyl or amino.

15. A compound of formula (X) 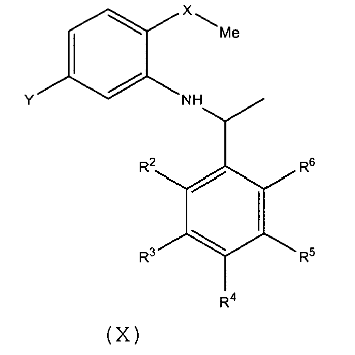 wherein
**Y** is halo;
**X** is -S-;
**R²,R³, R⁴, R⁵, and R⁶** are, each independently, hydrogen; halo; C₁₋₃alkoxy; or two adjacent R², R³, R⁴, R⁵, and R⁶ together with the two carbon atoms of the benzene ring to which they are attached form the ring 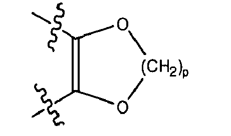 wherein **p** is 2, 3, 4, 5, or 6.

16. A compound of formula (VI) 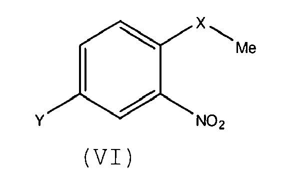 wherein
**Y** is 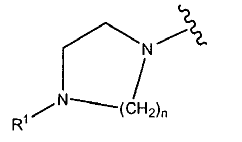
**R¹** is hydrogen, C₁₋₆alkyl optionally substituted with C₃₋₇cycloalkyl, phenyl optionally substituted with C₁₋₆alkyl or C₁₋₆alkoxy, -C(=O)-R⁷, or a protecting group;
**n** represents zero, 1, 2, 3, or 4;
**X** is -SO₂-; and
**R⁷** is C₁₋₆alkyl or amino.

17. The process according to claim 1, wherein the methyl nucleophile is methyl magnesium halide or methyl lithium.

18. the process according to claim 1, wherein the organic solvent is selected from tetrahydrofuran, heptanes, dioxane, and toluene.

19. The process according to claim 1, wherein the temperature is between -100 and -40° C.

20. The process according to any one of claims 2-4, wherein the dehydrating conditions are the azeotropic removal of water from an organic solvent.

21. The process according to claim 20, wherein the organic solvent is toluene or tetrahydrofuran.

22. The process according to any one of claims 2-4 wherein the drying agent is selected from anhydrous MgSO₄, anhydrous NaSO₄, and molecular sieves.

23. The process according to any one of claims 2-4, wherein the catalyst is selected from camphorsulphonic acid, SiO₂, TiCl₄, and p-toluenesulphonic acid.

24. Use of the compound of formula (IX) according to claim 14, as an intermediate in the preparation of a compound of formula (X), (Xa), (Xb), or (Xc), salts, and solvates thereof as defined in any one of claims 1 or 6-9.

25. Use of the compound of formula (X) according to claim 15, as an intermediate in the preparation of a compound of formula (Xa), (Xb, or (Xc), salts, and solvates thereof as defined in any one of claims 6-9.

26. Use of the compound of formula (VI) according to claim 16, as an intermediate in the preparation of a compound of formula (X), (Xa), (Xb), or (Xc), salts, and solvates thereof as defined in any one of claims 1 or 6-9.
